# FASCICULE DE BREVET EUROPEEN

(11) **EP 0 625 202 B1**
(45) Date de publication et mention de la délivrance du brevet: **28.07.1999**
(21) Numéro de dépôt: 93904116.6
(22) Date de dépôt: 26.01.1993
(51) Int. Cl.: C12N 15/14, C12N 15/81, C12P 21/02, C07K 14/00, A61K 38/00

(54) **SERUM-ALBUMINE HUMAINE, PREPARATION ET UTILISATION**
HUMANES SERUM ALBUMIN, HERSTELLUNG UND VERWENDUNG
SERUM-HUMAN ALBUMIN, PREPARATION AND UTILIZATION

(30) Priorité: 27.01.1992 FR 9200807
(43) Date de publication de la demande: 23.11.1994
(73) Titulaire: RHONE-POULENC RORER S.A., 92160 Antony (FR)
(72) Inventeur: BECQUART, Jérôme, F-75015 Paris (FR); FLEER, Reinhard, F-91440 Bures-sur-Yvette (FR); JUNG, Gérard 12, rue des Grands-Jardins, F-91310 Montlhéry (FR)
(74) Mandataire: Le Pennec, Magali
(86) Numéro de dépôt international: FR9300072
(87) Numéro de publication internationale: WO9315204

(56) Documents cités:
- EP-A- 0 344 459
- EP-A- 0 424 117
- EP-A- 0 464 590
- WO-A-93/00437
- FR-A- 2 635 115

## Description

La présente invention concerne une préparation de sérum-albumine humaine, un procédé pour son obtention, et ses utilisations.

La sérum-albumine humaine (SAH) est une protéine monomérique non glycosylée de 585 acides aminés, d'un poids moléculaire de 66 KD. Sa structure globulaire est maintenue par 17 ponts disulfures qui créent une série séquentielle de 9 boucles doubles (Brown, J.R., "Albumin Structure, Function and Uses", Rosenoer, V.M. et al. (eds.) Pergamon Press, Oxford, (1977) 27-51). Les gènes codant pour la SAH sont connus pour être hautement polymorphes, et plus de 30 variants génétiques apparemment différents ont été repérés par analyse électrophorétique dans des conditions variées (Weitkamp, L.R. et al., Ann. Hum. Genet. 37 (1973) 219-226). Le gène de la SAH est coupé en 15 exons par 14 séquences introniques et comprend 16 961 nucléotides, du site de "capping" supposé jusqu'au premier site d'addition de poly(A).

L'albumine humaine est synthétisée dans les hépatocytes du foie, puis sécrétée dans le flux sanguin. Cette synthèse conduit dans un premier temps à un précurseur, la prépro-SAH, qui contient une séquence signal de 18 acides aminés dirigeant le polypeptide naissant dans la voie de sécrétion.

La SAH est la protéine la plus abondante du sang, avec une concentration d'environ 40 g par litre de sérum. Il y a donc environ 160 g d'albumine circulante dans le corps humain à tout moment. Le rôle le plus important de la SAH est de maintenir une osmolarité normale du flux sanguin. Elle présente également une capacité exceptionnelle de liaison pour diverses substances et joue un rôle aussi bien dans le transport endogène de molécules hydrophobes (tels que les stéroïdes et les sels biliaires) que dans celui de différentes substances thérapeutiques qui peuvent ainsi être transportées à leurs sites d'action respectifs. De plus, la SAH a été récemment impliquée dans le catabolisme des prostaglandines.

La SAH représente 40 % du marché mondial des protéines plasmatiques. Son intérêt commercial réside dans le fait que ce produit est largement utilisé, par exemple dans des solutés dits de remplissage pour compenser les pertes de sang au cours d'actes chirurgicaux, d'accidents ou d'hémorragies, et à des doses pouvant atteindre plusieurs dizaines de grammes par jour et par individu. Actuellement, la consommation annuelle de SAH peut être estimée à plus de 300 tonnes.

Jusqu'à présent, la SAH disponible sur le marché est produite par purification à partir de matériel biologique d'origine humaine. En particulier, elle est obtenue par les techniques classiques de fractionnement du plasma provenant de dons de sang (Cohn et al., J. Am. Chem. Soc. 68 (1946) 459 pp), ou par extraction à partir du placenta humain, selon la technique décrite par J. Liautaud et al. (13ème Congès International d'IABS, Budapest; A: "Purification of proteins. Development of biological standard", Karger (ed.), Bale, 27 (1973) 107 pp).

Le développement du génie génétique et des nouvelles techniques d'extraction et de purification a ouvert la possibilité d'obtenir, à un prix de revient plus faible, des produits améliorés de plus haute pureté, de meilleure stabilité et sans risque de contamination virale (par exemple hépatite B et SIDA). Compte tenu de l'importance du marché de la SAH, la possibilité de produire cette protéine par voie recombinante a été largement étudiée. Ainsi, de nombreux systèmes d'expression ont été étudiés pour la préparation de la SAH recombinante.

Plus particulièrement, en ce qui concerne les hôtes bactériens, les premières expériences de génie génétique ont utilisé la bactérie E.coli comme organisme hôte. Ainsi, les brevets européens EP 236 210, EP 200 590, EP 198 745, ou EP 1 929 décrivent des procédés de production de la SAH chez E.coli en utilisant différents vecteurs d'expression, différents promoteurs transcriptionnels, et différents signaux de sécrétion. Par la suite, des travaux concernant la sécrétion de la SAH dans Bacillus subtilis ont également été réalisés, même si les niveaux d'albumine obtenus dans ce système ne semblent pas encore satisfaisants (Saunders et al., J. Bacteriol. 169 (1987) 2917).

En ce qui concerne les hôtes eucaryotes, des procédés pour la production de la SAH ont été mis au point en utilisant des levures comme organisme hôte. Ainsi, la sécrétion de la SAH dirigée par son propre peptide signal sous le contrôle du promoteur de la chélatine a pu être mise en évidence dans S.cerevisiae (Etcheverry et al., Bio/Technology 4 (1986) 726). La production de la SAH a également été mentionnée dans la levure de brasserie lors de la fabrication de la bière, en utilisant un procédé post-fermentaire (EP 201 239). Plus récemment, la demande de brevet EP 361 991 décrit un système particulièrement performant utilisant la levure Kluyveromyces comme organisme hôte, transformée avec des vecteurs dérivés du plasmide pKD1. Des niveaux particulièrement élevés de SAH sécrétée dans le milieu de culture ont pu être obtenus avec ce système. Enfin, la production de SAH recombinante a également été décrite dans Pichia pastoris, (EP 344 459). En outre, la purification de la SAH a également fait l'objet de nombreuses études (EP 319 067).

Pourtant, en dépit des efforts importants consacrés à l'obtention de la SAH par voie recombinante, ce produit n'est pas encore présent sur le marché. Ceci est lié à la difficulté de mettre au point un procédé assez performant basé sur le génie génétique, permettant d'obtenir, à l'échelle industrielle et dans des conditions économiquement rentables une SAH utilisable au niveau pharmaceutique. En particulier, si les questions de productivité semblent à peu près résolues (production à des niveaux élevés d'une albumine sécrétée, correctement maturée, et possédant une structure tertiaire conforme à celle de la sérum-albumine humaine native), les procédés décrits dans l'art antérieur ne permettent pas d'obtenir une SAH utilisable au niveau pharmaceutique. Il est en effet indispensable que la SAH produite soit conforme à certaines normes qualitatives. En particulier, compte tenu de son usage pharmaceutique, la SAH recombinante doit posséder les propriétés physico-chimiques de l'albumine native et respecter certains critères d'homogénéité, de pureté, et de stabilité. Ainsi, la pharmacopée fixe un certain nombre de paramètres pour les solutions d'albumine plasmatique, à savoir une valeur de pH, une teneur en protéines, une teneur en polymères et agrégats, une teneur en phosphatase alcaline, en potassium, en sodium et une certaine composition en protéines. Elle impose de plus une certaine absorbance, la conformité à un test de stérilité, à un essai de pyrogènes et de toxicité (voir "Albumini humani solutio" pharmacopée Européenne (1984) 255).

L'un des problèmes majeurs des procédés de l'art antérieur faisant appel aux techniques du génie génétique, est qu'ils génèrent une SAH recombinante colorée. Ce phénomène est tout particulièrement important dans le cas de systèmes permettant l'expression et la sécrétion de la SAH recombinante dans le milieu de culture. A cet égard, les exemples B1 et B2 de la présente demande illustrent le problème de la coloration dans le cas d'un système d'expression mettant en oeuvre, dans les conditions de l'art antérieur, une levure comme organisme hôte. L'exemple B1 n'est pas restrictif mais illustre un phénomène de coloration qui a été observé pour toutes les souches testées, quel que soit le mode de fermentation adopté (fed-batch, batch, continu). Par ailleurs, en dépit des efforts nombreux effectués dans ce but, cette coloration n'a jamais pu être éliminée par purification (voir exemple B2). Dans ces conditions, il n'a jamais été décrit dans l'art antérieur de SAH produite par voie recombinante, qui soit dépourvue de cette coloration. Or la présence de cette coloration constitue un obstacle à l'exploitation pharmaceutique de la SAH recombinante. En effet, les produits ainsi obtenus sont hétérogènes puisqu'ils comprennent les composants responsables de la coloration. De plus, leur composition est indéfinie puisque la coloration peut varier d'une préparation à une autre et que l'art antérieur ne permet pas de contrôler cette coloration. Dans ces conditions, il est très difficile de définir un procédé reproductible, permettant d'obtenir à chaque fois des préparations identiques de SAH recombinante. Enfin, en raison de la présence des composants responsables de la coloration, la SAH recombinante de l'art antérieur est potentiellement immunogène.

L'un des aspects de l'invention est de fournir une préparation de SAH recombinante de bonne qualité, possédant les propriétés de la SAH d'extraction, et utilisable dans le domaine pharmaceutique.

En particulier, un aspect de l'invention est de fournir une préparation de SAH recombinante possédant, après purification par les méthodes connues (concentration, précipitation, chromatographies, etc), un indice de colorimétrie inférieur à 0,2. Au sens de la présente invention, on entend par indice de colorimétrie le rapport de l'absorbance à 300 nm sur l'absorbance à 280 nm (i = DO₃₀₀ / DO₂₈₀). Ce rapport caractérise particulièrement bien la colorimétrie de la SAH puisqu'il reflète, pour une solution donnée, son absorbance indépendemment de sa concentration.

Un objectif de l'invention est de permettre l'obtention en quantités industrielles et à un niveau économiquement rentable d'une préparation de SAH recombinante utilisable pharmaceutiquement.

La demanderesse a maintenant montré qu'il est possible d'obtenir une solution de SAH non colorée en quantités industrielles par voie recombinante. La présente invention repose sur la mise en évidence que la qualité de la SAH produite n'est pas uniquement liée à l'hôte ou au vecteur choisi, ou au procédé de purification de la SAH à partir du milieu, mais en grande partie à la composition même du milieu de production. Ainsi, en modifiant notamment les sources carbonées et les conditions de préparation du milieu de production, des solutions de SAH recombinante possédant un indice de colorimétrie inférieur à 0,2 ont pu être obtenues.

Un premier objet de l'invention réside donc dans une sérum-albumine humaine caractérisée en ce qu'elle possède un indice de colorimétrie inférieur à 0,2 et en ce qu'elle résulte de l'expression dans un hôte eucaryote ou procaryote d'une séquence d'ADN exogène.

La présente invention fournit pour la première fois une SAH recombinante ayant un indice de colorimétrie inférieur à 0,2. Elle fournit ainsi une SAH recombinante utilisable dans le domaine pharmaceutique, avec des risques très faibles de réactions immunogènes. De plus, par rapport à la SAH plasmatique, la SAH de l'invention offre l'avantage d'être homogène et de composition parfaitement définie. En effet, en raison de son polymorphisme, de nombreux variants de la SAH existent. Ainsi, parmi ceux qui ont été identifiés, certains variants conservent un peptide pro substitué (Brennen et Carrell, Nature 274 (1978) 908; Galliano et al., Rev. Fr. Transfus. Immuno-Hématol. 27 (1984) 597), d'autres possèdent des mutations ponctuelles (Winter et al., Biochemistry 11 (1972) 889; Franklin et al., Proc.Natl.Acad.Sci. USA 77 (1980) 2505; Brennan, Biochim.Biophys.Acta 830 (1985) 320; Galliano et al., Protides Biol. Fluids Proc. Colloq. 34 (1986) 815; Takahashi et al., J. Chromatogr. 359 (1986) 181) ou des délétions au niveau de l'extrémité C-terminale (Galliano et al., J. Biol. Chem. 261 (1986) 4283). De plus, nombreux sont les variants dont les changements structuraux n'ont pas été identifiés. De ce fait, la SAH plasmatique obtenue par extraction de matériel biologique issu d'un très grand nombre de donneurs humains renferme potentiellement tous les variants de la SAH résultant de son polymorphisme. La présente invention fournit une SAH homogène et définie, en raison de sa préparation par voie génétique, par expression d'une ou plusieurs séquences d'ADN identifiées.

Préférentiellement, la SAH de la présente invention possède un indice de colorimétrie inférieur à 0,15.

Encore plus préférentiellement, la SAH de la présente invention possède un indice de colorimétrie inférieur à 0,1.

D'autres caractéristiques physico-chimiques de la SAH de la présente invention sont données dans l'exemple B5, à savoir notamment son spectre de fluorescence. L'ensemble de ces paramètres témoigne de la qualité de la SAH de l'invention.

Au sens de la présente invention, on entend par séquence d'ADN exogène toute séquence d'ADN introduite artificiellement dans l'hôte utilisé et codant pour la SAH. En particulier, il peut s'agir, selon l'hôte, de séquences génomiques, d'ADNc, de séquences hybrides, etc. Pour une meilleure mise en oeuvre de l'invention, on préfère cependant utiliser un ADNc. De telles séquences ont déjà été décrites dans l'art antérieur (Cf notamment EP 361 991 et Dugaiczyk et al., J. Supramol. Struct. & Cell Biochem., Suppl. 5 (1981)). Par ailleurs, cet ADN exogène comprend généralement une région de démarrage de la transcription et de la traduction jointe à l'extrémité 5' terminale de la séquence codante, de façon à diriger et à réguler la transcription et la traduction de ladite séquence. Le choix de ces régions promotrices peut varier en fonction de l'hôte utilisé.

Dans le cadre de la présente invention, l'ADN exogène fait préférentiellement partie d'un vecteur, qui peut être à réplication autonome ou intégratif. Plus particulièrement, des vecteurs à réplication autonome peuvent être préparés en utilisant des séquences à réplication autonome chez l'hôte choisi. A titre d'exemple, chez la levure, il peut s'agir d'origines de réplication dérivées de plasmides: pKD1 (EP 241 435), 2µ (Beggs, Nature 275 (1978) 104-109), etc; ou bien de séquences chromosomiques (ARS). S'agissant des vecteurs intégratifs, ceux-ci peuvent être préparés par exemple en utilisant des séquences homologues à certaines régions du génome de l'hôte, permettant, par recombinaison homologue, l'intégration du vecteur. A cet égard, l'utilisation d'ADNr permet une intégration multiple de l'ADN exogène, et donc sa présence en plus grand nombre de copies par cellules.

Dans un mode préféré, la SAH de l'invention résulte de l'expression dans un hôte eucaryote ou procaryote d'une séquence d'ADN exogène et de la sécrétion du produit d'expression de ladite séquence dans le milieu de culture. Il est en effet particulièrement avantageux de pouvoir obtenir par voie recombinante une SAH de qualité pharmaceutique directement dans le milieu de culture. Dans ce cas, la séquence d'ADN exogène comprend, en amont de la séquence codant pour la SAH, ou, le cas échéant, entre la région de démarrage de la transcription et de la traduction et la séquence codante, une séquence "leader" dirigeant la protéine naissante dans les voies de sécrétion de l'hôte utilisé. Cette séquence "leader" peut être la séquence "leader" naturelle de la SAH, mais il peut également s'agir d'une séquence hétérologue (issue d'un gène codant pour une autre protéine) ou même artificielle. Le choix de l'une de ces séquences est notamment guidé par l'hôte utilisé. A titre d'exemple, lorsque l'hôte utilisé est une levure, il est possible d'utiliser comme séquence "leader" hétérologue celle de la phéromone facteur α, de l'invertase ou de la phosphatase acide.

Parmi les hôtes eucaryotes utilisables dans le cadre de la présente invention, on peut citer les cellules animales, les levures, ou les champignons. En particulier, s'agissant de levures, on peut citer les levures du genre Saccharomyces, Kluyveromyces, Pichia pastoris, Schwanniomyces, ou Hansenula. S'agissant de cellules animales, on peut citer les cellules COS, CHO, C127, etc. Parmi les champignons susceptibles d'être utilisés dans la présente invention, on peut citer plus particulièrement Aspergillus ssp. ou Trichoderma ssp.

Comme hôtes procaryotes, on préfère utiliser les bactéries suivantes E.coli, Bacillus, ou Streptomyces.

Un autre objet de l'invention concerne un procédé de préparation de SAH ayant un indice de colorimétrie inférieur à 0,2 selon lequel on réalise les étapes suivantes :
- dans une première étape, on introduit dans une cellule hôte eucaryote ou procaryote un ADN exogène codant pour la sérum-albumine humaine sous contrôle de signaux de transcription et de traduction appropriés à l'hôte utilisé,
- dans une deuxième étape, on cultive la cellule ainsi obtenue dans un milieu de composition définie contenant au moins une source carbonée choisie parmi les alcools, les sucres non-réducteurs, les acides organiques ou les dérivés du glucose substitués sur l'oxygène du carbone C4; ou dans un milieu préparé de façon à éliminer ou à limiter la formation d'impuretés de type aldéhydique, et,
- dans une troisième étape, on récupère la SAH produite.

Plus particulièrement, lors de la première étape du procédé de l'invention, l'ADN exogène peut être introduit dans la cellule hôte par différentes techniques. Notamment, l'introduction peut être effectuée par transformation, conjugaison, ou électroporation.

S'agissant de la transformation, différents protocoles ont été décrits dans l'art antérieur. En particulier, elle peut être réalisée en traitant les cellules entières en présence d'acétate de lithium et de polyéthylène glycol selon la technique décrite par Ito et al. (J. Bacteriol. 153 (1983) 163-168), ou en présence d'éthylène glycol et de diméthylsulfoxyde selon la technique de Durrens et al. (Curr. Genet. 18 (1990) 7). Un protocole alternatif a également été décrit dans la demande de brevet EP 361 991. Plus spécifiquement, pour les cellules procaryotes, la transformation peut être effectuée selon la technique décrite par Dagert at al. (Gene 6 (1979) 23-28) par traitement avec une solution de CaCl₂ puis choc thermique. Pour les cellules animales, elle peut également être réalisée par la technique au phosphate de calcium selon Haynes (Nucleic Acids Res., 11 (1983) 687-706).

S'agissant d'électroporation, la technique décrite par Karube et al. (FEBS Letters 182 (1985) 90) peut avantageusement être utilisée.

Le choix de l'une ou de l'autre de ces méthodes est établi notamment en fonction de l'hôte choisi et de l'ADN exogène utilisé.

Parmi les hôtes eucaryotes utilisables dans le procédé de l'invention, on peut citer les cellules animales, les levures, ou les champignons qui ont été mentionnés ci-avant. Parmi les hôtes procaryotes, on peut utiliser toute bactérie définie plus haut.

Dans un mode préféré de l'invention, le procédé est mis en oeuvre en utilisant comme cellule hôte une cellule eucaryote.

Encore plus préférentiellement, le procédé de l'invention est mis en oeuvre en utilisant comme cellule hôte une levure.

L'ADN exogène codant pour la SAH utilisable dans le procédé est défini comme précédemment. Préférentiellement, il s'agit d'un ADNc, d'un ADN génomique ou d'un ADN hybride. Pour une meilleure mise en oeuvre de l'invention, on préfère cependant utiliser un ADNc. Par ailleurs, cet ADN exogène comprend généralement une région de démarrage de la transcription et de la traduction jointe à l'extrémité 5' terminale de la séquence codante, de façon à diriger et à réguler la transcription et la traduction de ladite séquence. Le choix de cette région peut varier en fonction de l'hôte utilisé.

Dans un mode particulièrement avantageux de l'invention, l'ADN exogène comprend, en amont de la séquence codant pour la SAH mature, une séquence "leader" dirigeant la protéine naissante dans les voies de sécrétion de l'hôte utilisé. De telles séquences ont été définies plus haut. Par ailleurs, dans le cadre de la présente invention, l'ADN exogène fait préférentiellement partie d'un vecteur, qui peut être à réplication autonome ou intégratif, tel qu'indiqué plus haut.

Dans un mode préféré de l'invention, le procédé de production est caractérisé en ce que la SAH est sécrétée dans le milieu de culture. Il est en effet particulièrement avantageux de pouvoir obtenir par voie recombinante une SAH de qualité pharmaceutique directement dans le milieu de culture.

La seconde étape du procédé de l'invention consiste à cultiver les cellules recombinées dans des conditions permettant l'expression de la séquence d'ADN exogène codant pour la SAH. Cette étape est particulièrement importante puisqu'elle influence directement la quantité et la qualité de la SAH produite. La présente invention décrit pour la première fois des conditions de culture permettant la production d'une sérum-albumine de qualité pharmaceutique.

Parmi les alcools utilisables dans le procédé de l'invention, on peut citer préférentiellement les alcools simples comportant au moins 2 atomes de carbone (éthanol, etc), ou les polyalcools (glycérol, sorbitol, etc). Comme sucres non-réducteurs, on peut citer par exemple le saccharose, et comme acides organiques les acétates ou les lactates. Les dérivés du glucose utilisables dans la présente invention répondent plus précisément à la formule suivante : 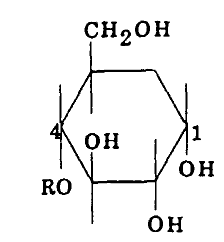 dans laquelle R est différent de l'hydrogène. A titre d'exemple on peut citer les disaccharides, et de préférence les disaccharides présentant une liaison osidique de type 1-4 tels que le maltose, le cellobiose, ou le lactose.

Il est entendu que le choix de l'un ou de plusieurs de ces composés est guidé par l'hôte utilisé. Toutefois, il est également possible de modifier un hôte pour le rendre capable d'assimiler une source carbonée préférée.

Ces différentes sources carbonées peuvent être utilisées séparément ou en association. Par exemple, l'association d'un dérivé du glucose et d'un alcool donne de très bons résultats. De même, l'association d'un alcool simple et d'un polyalcool donne également une albumine de très haute qualité. En outre, un résultat inattendu est que ce type d'association permet également, dans certains cas, d'augmenter les niveaux de production de la SAH, et donc d'améliorer les rendements du procédé de fabrication.

Le procédé de l'invention peut également être mis en oeuvre dans un milieu préparé de façon à éliminer ou à limiter la formation d'impuretés de type aldéhydiques. Ce type de préparation est généralement inutile lorsque l'on utilise, dans un milieu de composition définie, une ou plusieurs des sources carbonées énoncées ci-avant. Différents moyens peuvent être employés pour limiter la formation de telles impuretés, dont le choix dépend du milieu (nature de la source carbonée) et de l'hôte considérés. A titre d'exemple, il peut être avantageux de stériliser la source carbonée à froid (par exemple par filtration comme illustré dans l'exemple B4).

La troisième étape de l'invention permet d'extraire du milieu de culture la SAH produite. Dans le cas où la SAH est sécrétée dans le milieu par les cellules recombinées, cette extraction peut se faire directement à partir du surnageant de culture obtenu par centrifugation. Dans le cas où la SAH n'est pas sécrétée, il est nécessaire, préalablement à l'extraction, de casser les cellules en culture afin de libérer la SAH qu'elles contiennent. Cette étape préalable peut être réalisée par différents moyens physiques ou physico-chimiques (sonication, broyage, choc osmotique, choc thermique, etc).

L'extraction peut être réalisée par différentes techniques connues de l'homme du métier et décrites dans la littérature. Généralement, ces techniques font appel à des étapes de concentration, de précipitation et de chromatographie. Certaines de ces différentes techniques sont illustrées dans les exemples.

Un autre objet concerne toute composition pharmaceutique comprenant de la SAH telle que décrite ci-dessus.

La présente invention sera plus complètement décrite à l'aide des exemples qui suivent, qui doivent être considérés comme illustratifs et non limitatifs.

### LEGENDE DES FIGURES

Figure 1 : Construction du promoteur hybride PGK/GAL. P = promoteur; UAS = "Upstream Activating Sequence".

Figure 2: Stratégie de construction et représentation du vecteur pYG401. P = promoteur; T = Terminateur de transcription; bla = gène conférant la résistance à l'ampicilline; aph = gène conférant la résistance à la généticine (G418).

Figure 3: Construction et représentation du plasmide pP4-33.

Figure 4: Construction et représentation du plasmide pYG65.

Figure 5: Construction et représentation du plasmide pYG70.

Figure 6: Construction et représentation du plasmide pYG72

Figure 7: Construction et représentation du plasmidepYG404ΔHindIII.

Figure 8: Construction et représentation du plasmide pYG128.

Figure 9: Construction et représentation des plasmides pYG131 et pYG132.

Figure 10: Carte de restriction du plasmide pYG600. bla = gène de la β-lactamase conférant la résistance à l'ampicilline.

Figure 11: Stratégie de construction du plasmide pYG70-2. aph = gène de la 3'-aminoglycoside phosphotransférase conférant la résistance à la généticine (G418); prom = promoteur; IR = séquence répétée inversée; ORI = origine de réplication; LacZ' = gène de structure de la β-galactosidase. Les sites marqués d'une (∗) possèdent des extrêmités compatibles avec les sites correspondants sans reconstituer, après ligation, de sites de coupure reconnus par lesdites enzymes.

Figure 12: Séquence des oligodésoxynucléotides de synthèse A-F ayant servi à la construction des adaptateurs 1 à 3.

Figure 13: Stratégie de construction du plasmide pYG70-3. Pour la légende, voir figure 11. term = terminateur

Figure 14 : Stratégie de construction du plasmide pYG1003. Pour la légende, voir figure 11.

Figure 15: Stratégie de construction du plasmide pYG1023. Pour la légende, voir figure 11.

Figures 16 et 17 : Spectres UV de différentes préparations d'albumine : Figure 16 : (1) albumine BCQ759 des exemple B1 et B2; (2) albumine BCQ804LE de l'exemple B5; (3) albumine témoin (IM). Figure 17 : albumine BCQ835GE de l'exemple B5.

Figures 18 et 19 : Spectres de fluorescence de différentes préparations d'albumine à 430 nm (figure 18) et 360 nm (figure 19).

### TECHNIQUES GENERALES DE CLONAGE

Les méthodes classiques de biologie moléculaire telles que la centrifugation d'ADN plasmidique en gradient de chlorure de césium - bromure d'éthidium, la digestion par les enzymes de restriction, l'électrophorèse sur gel, l'électroélution des fragments d'ADN à partir de gels d'agarose, la transformation dans E.coli, etc, sont décrites dans la littérature (Maniatis et al., "Molecular Cloning : a Laboratory Manual", Cold Spring Harbor Laboratory, Cold Spring Harbor, N.Y., 1986; Ausubel et al., (eds.), "Current Protocols in Molecular Biology", John Wiley & Sons, New York 1987).

La mutagénèse dirigée in vitro par oligodésoxynucléotides est effectuée selon la méthode développée par Taylor et al. (Nucleic Acids Res. 13 (1985) 8749-8764) en utilisant le kit distribué par Amersham. Le séquençage de nucléotides est réalisé selon la technique des didéoxy décrite par Sanger et al. (Proc. Natl. Acad. Sci. USA, 74 (1977) 5463-5467). L'amplification enzymatique de fragments d'ADN spécifiques est effectuée par réaction de PCR ("Polymerase-catalyzed Chain Reaction") dans les conditions décrites par Mullis et Faloona (Meth. Enzym., 155 (1987) 335-350) et Saiki et al (Science 230 (1985) 1350-1354), en utilisant un "DNA thermal cycler" (Perkin Elmer Cetus) en suivant les recommandations du fabricant.

### EXEMPLES

### A - Construction de vecteurs d'expression de la sérum-albumine humaine

### 1. Construction d'un vecteur d'expression de SAH sous contrôle d'un promoteur hybride PGK/GAL : plasmide pYG401.

Un vecteur d'expression de la sérum-albumine humaine a été préparé à partir du plasmide pYG19 (EP 361 991). Ce dernier comprend les éléments suivants:
- la séquence du plasmide pKD1, qui fait de pYG19 un plasmide à copies multiples, stable, et capable de se répliquer chez les levures du genre Kluyveromyces (EP 361 991).
- une cassette d'expression de la sérum-albumine humaine comportant le gène de structure codant pour la forme prépro sous contrôle du promoteur du gène PGK de S.cerevisiae;
- un réplicon et un marqueur de sélection (gène bla conférant la résistance à l'ampicilline) bactériens; et,
- le gène aph conférant la résistance au G418 à la levure.

Le vecteur pYG401 a été construit à partir du plasmide pYG19 par modification au niveau de la cassette d'expression de la sérum-albumine humaine. Dans pYG401, le gène de l'albumine n'est plus sous contrôle du promoteur du gène PGK de S.cerevisiae, mais sous contrôle d'un promoteur hybride entre les promoteurs des gènes PGK et GAL1/GAL10 de S.cerevisiae. Ce promoteur hybride a été obtenu en remplaçant la région UAS ("Upstream Activating Sequence") du promoteur PGK (Stanway et al., Nucl.Acid.Res. 15 (1987) 6855) par la région UAS du promoteur GAL1/GAL10 (Johnnton et Davies, Mol.Cell.Biol. 4 (1984) 1440; West et al., Mol.Cell.Biol. 4 (1984) 2467).

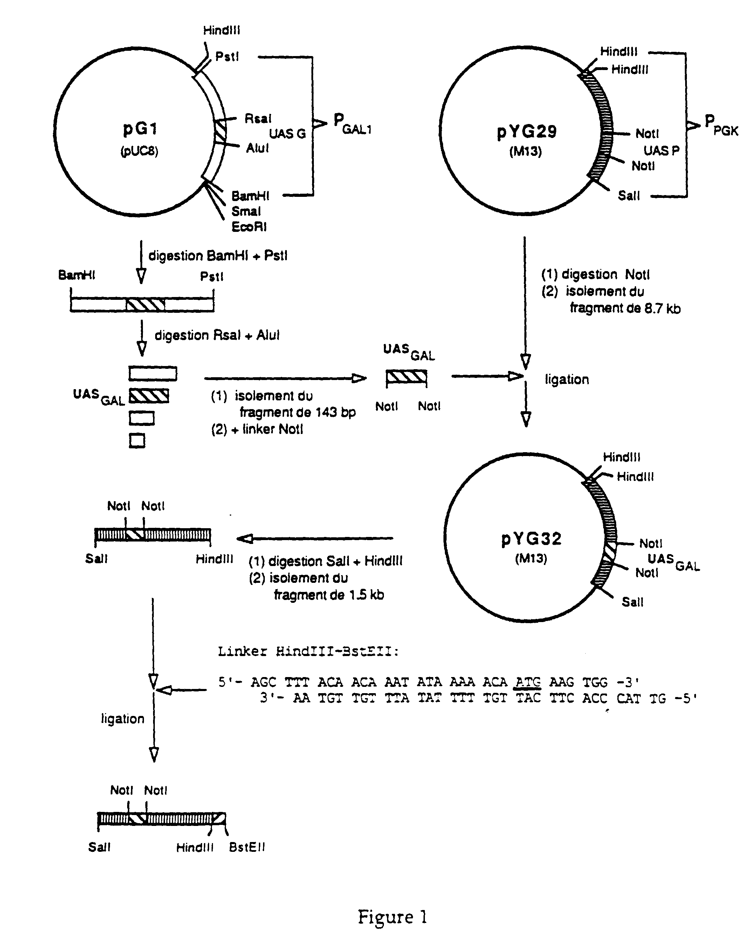

Ce promoteur hybride a été construit de la manière suivante (Cf figure 1):

L'obtention du plasmide pYG29 a été décrite en détail dans la demande EP361 991. Ce plasmide comporte le promoteur du gène PGK de S.cerevisiae isolé à partir du plasmide pYG19 sous forme d'un fragment SalI-HindIII, et cloné dans le bactériophage M13mp18, Il a ensuite été modifié par mutagénèse dirigée pour introduire les sites de restriction suivants :
- 1 site HindIII supplémentaire en position -25 par rapport à l'ATG. L'introduction de ce site permet de restaurer après les différentes étapes de clonage, une région nucléotidique proche de l'ATG identique à la région native du promoteur PGK. L'environnement du codon ATG est en effet connu pour influencer de manière sensible l'efficacité de l'initiation de la traduction des gènes eucaryotes (Kozak, M., Microbiol. Rev. 47 (1983) 1-45; Hamilton, R., Nucl.Acid.Res 15 (1987) 3581-3593).
- 2 sites Noti de part et d'autre de la région UAS.

L'UAS du promoteur GAL1/GAL10 a été isolée à partir du plasmide pG1 décrit par Miyajima et al. (Nucl. Acid. Res 12 (1984) 6397-6414; Cloning Vectors, Pouwels et al., Elsevier (1985) VI-B-ii-2). Ce plasmide est déposé à l'ATCC sous le numéro 37305.

Le plasmide pG1 comporte un fragment de 0,8 kb contenant le promoteur GAL1/GAL10 de S.cerevisiae, inséré dans le site HindII du plasmide pUC8, duquel il peut être excisé sous forme d'un fragment BamHI-PstI (figure 1).

Ce fragment a été excisé de pG1, purifié, puis digéré avec les enzymes RsaI et AluI, dont les sites de coupure respectifs sont localisés de part et d'autre de la région UAS. Un fragment de 143 pb a ainsi été isolé par electroélution, puis mis sous forme d'un fragment NotI en ajoutant un linker 5'-GCGGCCGC-3'. Ce fragment a ensuite été cloné dans le plasmide pYG29, préalablement digéré par NotI.

Le plasmide résultant est appelé pYG32 (figure 1).

Pour obtenir le vecteur d'expression portant ce promoteur hybride, le fragment SalI-HindIII portant le promoteur hybride a été isolé de pYG32 et ligaturé avec un adaptateur synthétique HindIII-BstEII composé des 2 brins complémentaires suivants : 5'-AGC TTT ACA ACA AAT ATA AAA ACA ATG AAG TGG-3' et 5'-GT TAC CCA CTT CAT TGT TTT TAT ATT TGT TGT AA-3' (le codon d'initiation de la transcription est représenté en caractères gras). Cet adaptateur reconstitue les 22 pb situées immédiatement en amont du gène de structure PGK de S.cerevisiae, et comprend les premiers codons du gène codant pour la préproHSA, jusqu'à un site BstEII présent dans le gène natif (figure 1).

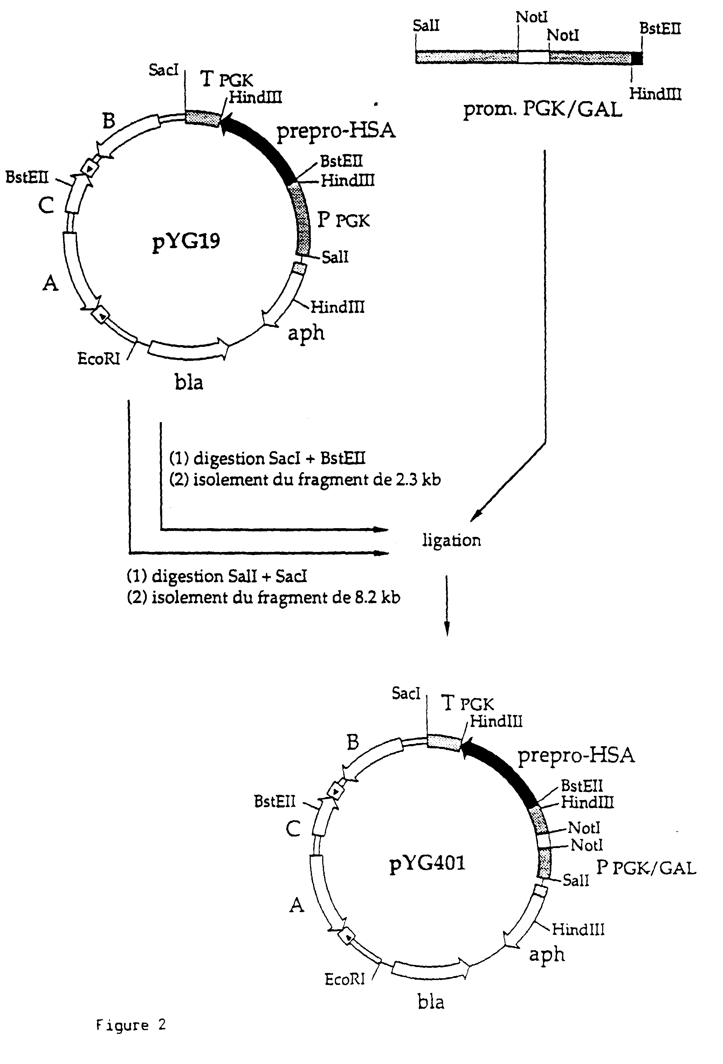

La cassette d'expression de l'albumine humaine a ensuite été reconstituée en ligaturant le fragment SalI-BstEII ainsi obtenu portant le promoteur hybride et l'extrêminté 5' du gène de structure de l'albumine, avec le fragment BstEII-SacI isolé du plasmide pYG19, portant le reste du gène de l'albumine, et le terminateur du gène PGK de S.cerevisiae (figure 2).

La cassette ainsi obtenue a été utilisée pour remplacer la cassette d'expression SalI-SacI portée par le plasmide pYG19.

Le vecteur résultant est appelé pYG401 (figure 2).

### 2. Construction d'un vecteur d'expression de SAH sous contrôle du promoteur K1ADH4 : plasmide pYG132.

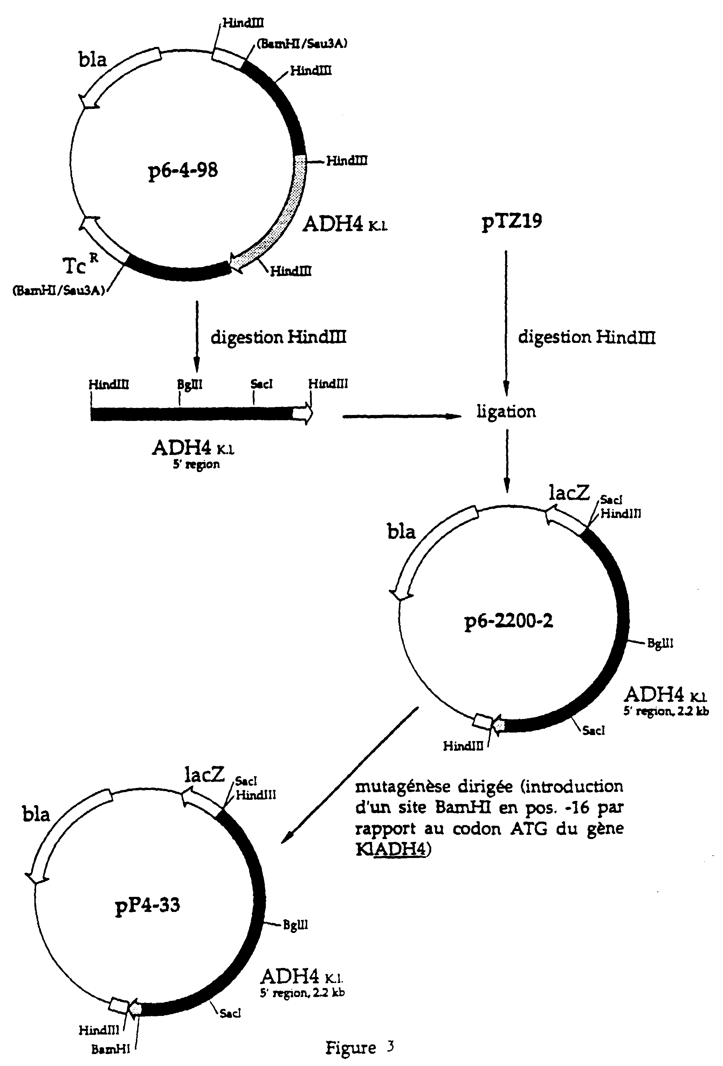

2.1. Isolement du promoteur K1ADH4 de K.lactis.
   Le promoteur K1ADH4 a été obtenu par criblage d'une banque d'ADN génomique total de Kluyveromyces lactis CBS2359/152 au moyen d'une sonde hétérologue provenant du gène ADH2 de S. cerevisiae. Plus précisément, la banque a été obtenue par clonage au site BamHI du vecteur de remplacement Lambda-L47 du produit d'une digestion partielle par l'enzyme Sau3A de l'ADN de K. lactis CBS2359/152 . La sonde utilisée pour l'hybridation est un fragment EcoRV-BamHI de 980 bp comprenant la région codante du gène de structure ADH2 de S. cerevisiae, à l'exception des 70 premières pb (sonde A). Ce fragment est obtenu par digestion enzymatique à partir d'un plasmide nommé pBR322.ADR2.BSa (Williamson et al., Cell 23 (1981) 605-614; Russell et al., J. Biol.Chem. 258 (1983) 2674-2682).
   Un fragment de 8 kb environ a ainsi été isolé et sous-cloné au site BamHI du plasmide pBR322 pour générer le plasmide p6-4-98 (figure 3). L'insert BamHI porté par ce plasmide a ensuite été cartographié au moyen d'enzymes de restriction, et la région promotrice du gène K1ADH4 a été localisée sur ce fragment par hybridations différentielles en utilisant la sonde A ainsi qu'une deuxième sonde correspondant au fragment BamHI-EcoRV de 1100 pb environ du plasmide pBR322.ADR2.BSa (sonde B).
   Dans une seconde étape, le plasmide p6-4-98 a été digéré par l'enzyme HindIII et un fragment de 2,2 kb a été isolé. Ce fragment a ensuite été purifié par des techniques standard et sous-cloné au site HindIII du plasmide pTZ19 pour générer le plasmide p6-2200-2 (figure 3). L'analyse du fragment sous-cloné révèle qu'il contient les 14 premiers codons du gène K1ADH4 et la région située en amont de celui-ci, comprenant les éléments de régulation de l'expression.
   La partie comprise entre le site BglII et le codon d'initiation de la traduction ATG (fragment de 1,2 kb environ) a été séquencée en utilisant la méthode de terminaison de chaine (Sanger et al., Proc.Nat.Acad.Sci. 74 (1977) 5463).
2.2. Construction d'un promoteur K1ADH4 portable (BglII-BamHI)
   Un promoteur portable a été préparé par insertion sur le fragment HindIII de 2,2 kb présent sur le plasmide p6-2200-2 d'un site de restriction BamHI en position - 16 par rapport au codon ATG du gène K1ADH4.
   L'insertion de ce site permet de générer un fragment BglII-BamHI de 1,2 kb comprenant exclusivement la région promotrice K1ADH4. Elle permet également d'introduire en aval du promoteur ainsi obtenu tout gène que l'on désire exprimer.
   Le site BamHI a été introduit en position - 16 par rapport au site d'initiation de la traduction (ATG) du gène K1ADH4 par mutagénèse dirigée en utilisant la technique de double amorce (Sambrook, Fritsch, Maniatis, Molecular Cloning Laboratory Manual, Cold Spring Harbor Lab Press, 1989). La séquence des oligodésoxynucléotides synthétiques utilisés pour cette mutagénèse est donnée ci-dessous. Le site BamHI généré est souligné, l'ATG est indiqué en italiques et les astérisques désignent les bases modifiées par rapport à la séquence initiale. SI = Séquence initiale; SM = Séquence modifiée. 
5'-CTCCCCCACCAACAACACAACATACAACACACGCAATGTTCAGATT-3' (SI)
** *
5'-CTCCCCCACCAACAACACAGGATCCAACACACGCAATGTTCAGATT-3' (SM)
3'-GAGGGGGTGGTTGTTGTGTCCTAGGTTGTGTGCGTTACAAGTCTAA-5' (SM)
 Le plasmide ainsi obtenu est appelé pP4-33 (figure 3).
2.3. Construction d'un vecteur d'expression de la sérum albumine humaine (HSA) :
   Pour construire un vecteur d'expression de la sérum-albumine humaine, un dérivé du plasmide pYG404 (Cf EP 361 991) a été préparé, contenant :
   - un réplicon de levure (séquence quasiment entière du plasmide naturel pKD1),
   - le gène codant pour la prépro-albumine humaine (HSA) sous contrôle du promoteur du gène LAC4 de K. lactis et suivi du terminateur du gène PGK de S. cerevisiae; le gène de structure codant pour la HSA est précédé par une séquence de 25 nucléotides correspondant à la région directement en amont du gène PGK de S. cerevisiae,
   - le gène aph conférant la résistance à la généticine (G418) à la levure, et
   - un réplicon et un marqueur de sélection (gène bla conférant la résistance à l'ampicilline) pour E. coli.

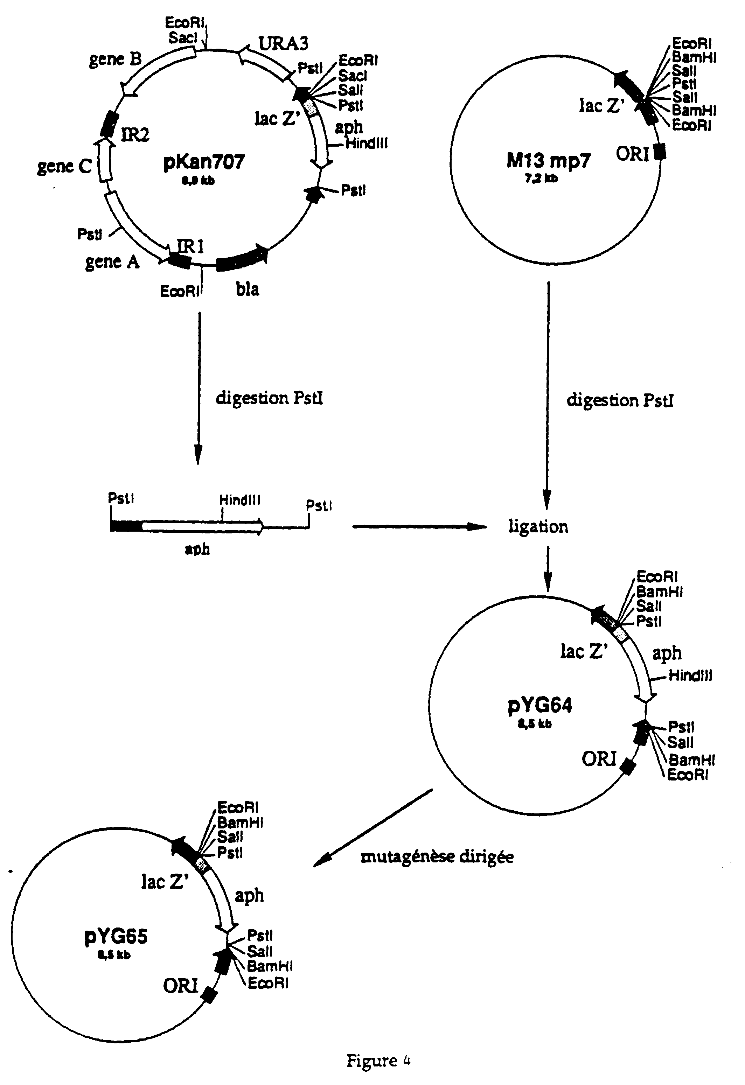

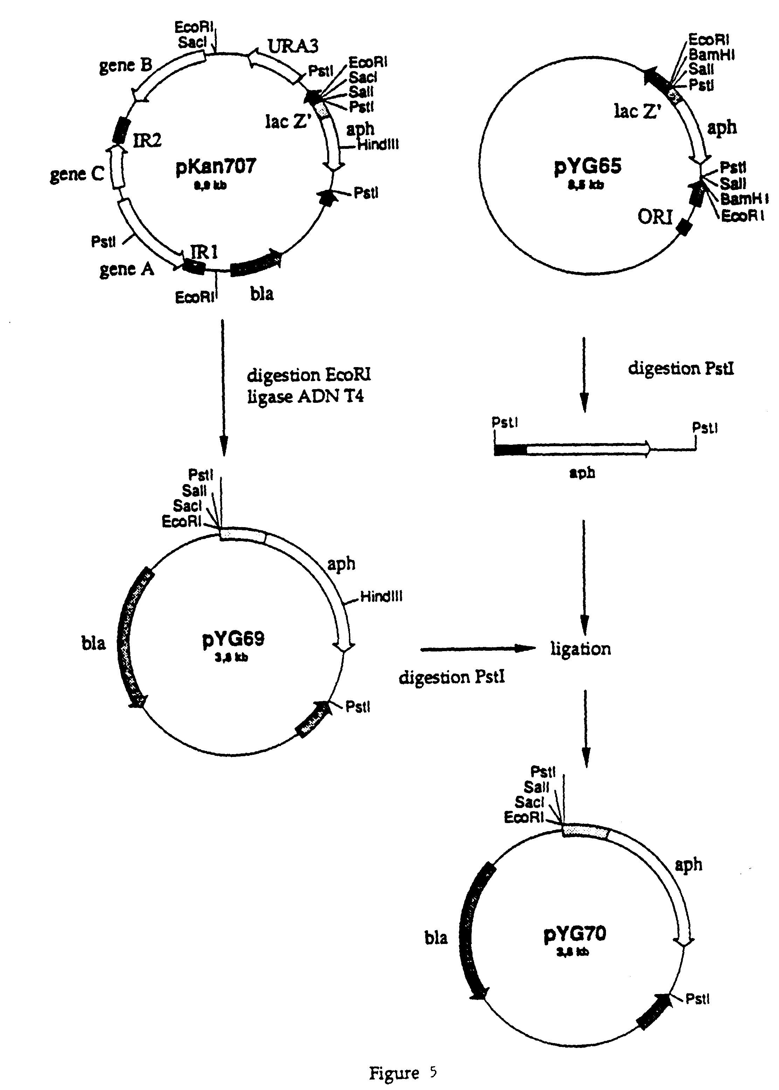

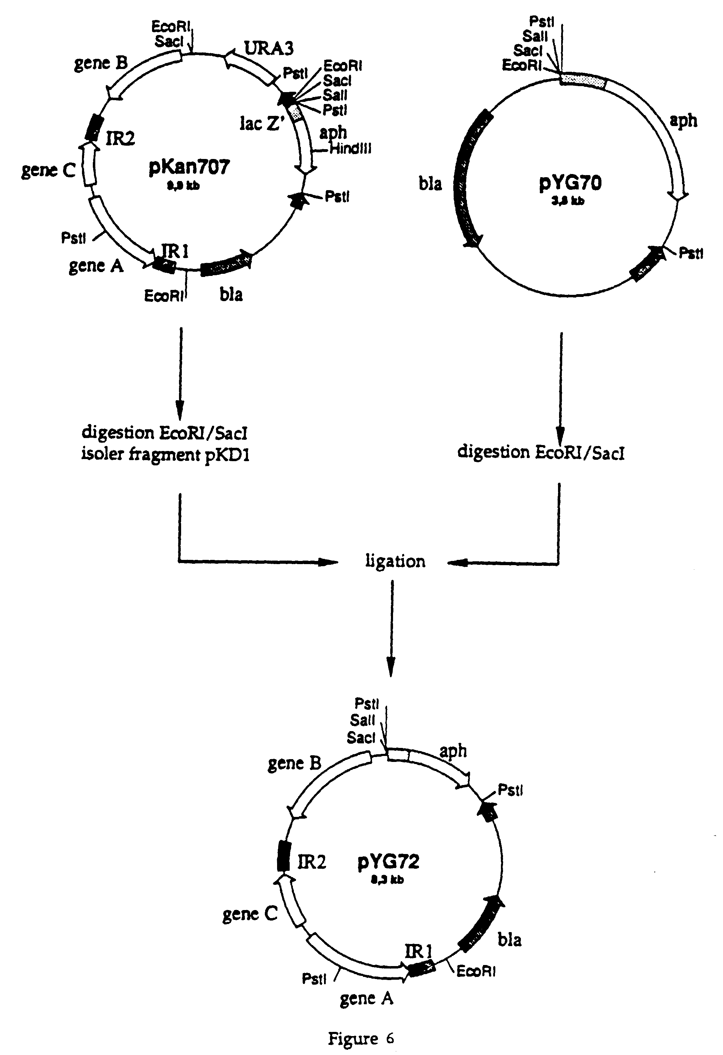

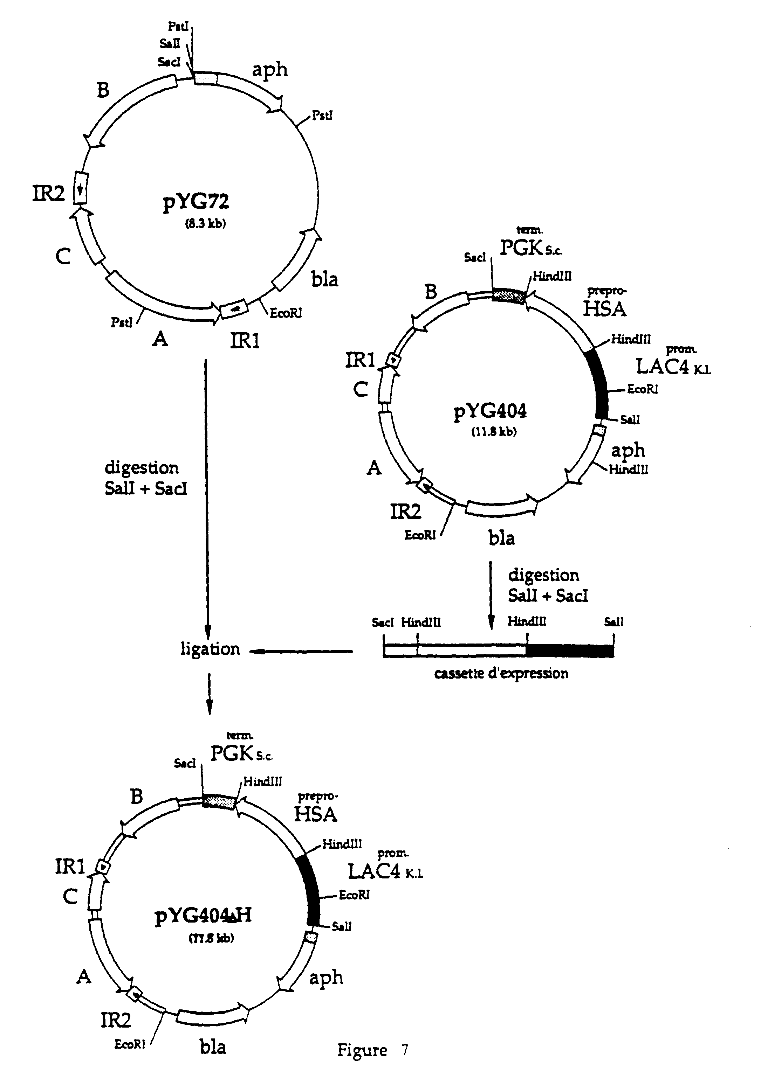

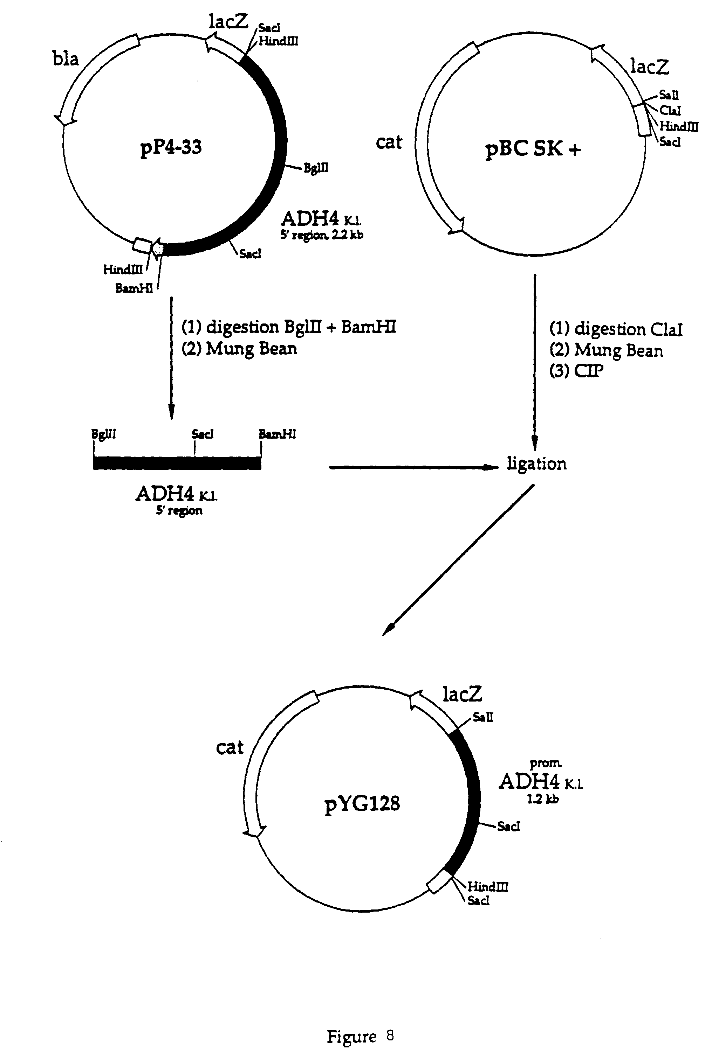

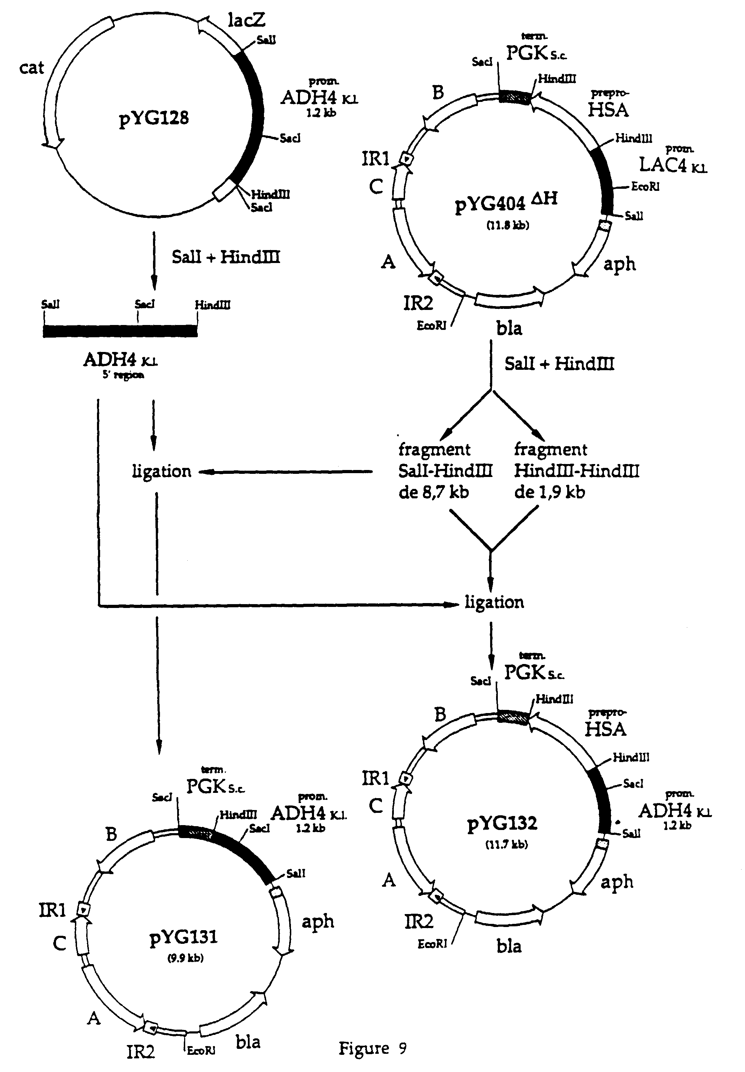

Ce plasmide, nommé pYG404ΔH, diffère de pYG404 uniquement par la destruction du site HindIII, localisé dans le gène aph, par mutagénèse dirigée. Cette modification a ensuite permis de substituer le promoteur LAC4 présent dans le plasmide pYG404ΔH sous forme d'un fragment SalI-HindIII par le promoteur K1ADH4 également construit sous forme d'un fragment SalI-HindIII.
(a) Construction du plasmide pYG404ΔH (figures 4-7).
   Pour effectuer la délétion du site HindIII dans le vecteur de clonage pYG404, différentes étapes de sous-clonage ont été effectuées, donnant lieu à une construction intermédiaire : pYG72 (figure 6). Ce vecteur correspond au plasmide pKan707 (EP 361 991) dans lequel le fragment SacI contenant le gène URA3 a été éliminé ainsi que le site unique HindIII présent dans le gène aph. Pour effectuer la mutagénèse dirigée sur ce site, le fragment PstI de 1,3 kb portant le gène aph a été sous-cloné à partir du plasmide pKan707 dans le bactériophage M13mp7 pour donner le vecteur pYG64 (figure 4). Le site HindIII a été détruit par mutagénèse dirigée (Cf techniques générales de clonage) en utilisant l'oligodésoxynucléotide suivant : 5'-GAA ATG CAT AAG CTC TTG CCA TTC TCA CCG-3', permettant le remplacement du triplet CTT codant pour la leucine 185 par le triplet CTC. Ce changement ne modifie pas la séquence protéique résultante. Le plasmide obtenu a été appelé pYG65 (figure 4). Pour construire le plasmide pYG72, la partie contenant le réplicon bactérien du vecteur pKan707 a été isolée par digestion avec l'enzyme EcoRI et recircularisation avec la T4 DNA ligase, générant le plasmide intermédiaire pYG69. Le fragment PstI présent dans celui-ci contenant le gène aph a ensuite été remplacé par le fragment équivalent muté provenant du plasmide pYG65. Cette construction a été appelée pYG70 (figure 5). La séquence de pKD1 de 4,7 kb comprise entre les sites EcoRI et SacI a ensuite été introduite dans ce vecteur pour obtenir pYG72 (figure 6).
   Le vecteur pYG404ΔH a été obtenu en insérant la cassette d'expression provenant du plasmide pYG404 (EP 361 991) sous forme d'un fragment SalI-SacI aux sites correspondants de pYG72 (figure 7).
(b) Construction d'un promoteur K1ADH4 portable [SalI-HindIII] (figure 8).
   Le promoteur K1ADH4 porté sur le fragment BglII-BamHI provenant du plasmide pP4-33 (Cf 2.1.) a été modifié de la manière suivante pour l'adapter à l'utilisation dans des vecteurs d'expression dérivés du plasmide pYG404ΔH:
   Après digestion du plasmide pP4-33 par les enzymes BglII et BamHI suivi d'un traitement à la nucléase 'Mung Bean' pour rendre les extrémités franches, le fragment de 1,2 kb portant le promoteur K1ADH4 a été isolé à partir d'un gel d'agarose et sous-cloné dans le vecteur pBC SK+ (Stratagene, La Jolla, CA, USA) préalablement linéarisé avec l'enzyme ClaI et traité à la nucléase 'Mung Bean' ainsi qu'à la phosphatase alkaline de veau (CIP). Le plasmide obtenu de cette manière (pYG128, figure 8) permet l'isolement du promoteur K1ADH4 sous forme d'un fragment SalI-HindIII de 1,2 kb.
(c) Construction du vecteur pYG132 (figure 9).
   La digestion du vecteur d'expression pYG404ΔH (Cf 2.3.(a)) par les enzymes SalI et HindIII permet le remplacement du promoteur LAC4 par le promoteur K1ADH4 décrit ci-dessus.
   Pour effectuer ce clonage, le fragment SalI-HindIII de 8,7 kb contenant la partie pKD1 et les marqueurs de sélection ainsi que le fragment HindIII-HindIII de 1,9 kb portant le gène codant pour la prépro-HSA ont été isolés à partir du vecteur pYG404ΔH et religués en présence du fragment SalI-HindIII de 1,2 kb provenant du plasmide pYG128 et portant le promoteur K1ADH4. Deux plasmides ont été obtenus de cette manière:
   - pYG131 (figure 9), correspondant à un vecteur de clonage permettant l'insertion au site HindIII unique de tout gène que l'on désire exprimer sous contrôle du promoteur K1ADH4, et
   - pYG132 (figure 9), qui est identique au plasmide pYG131 sauf qu'il contient le gène de la prépro-HSA introduit au site HindIII.

### 3. Construction d'un vecteur d'expression de SAH sous contrôle du promoteur du gène LAC4 de K.lactis : plasmide pYG1023.

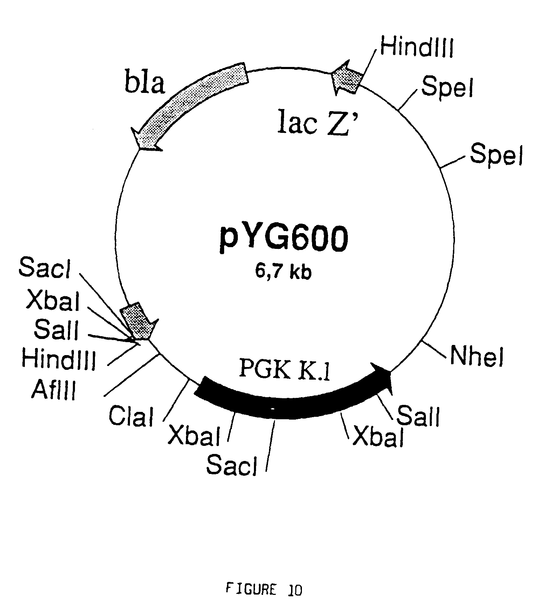

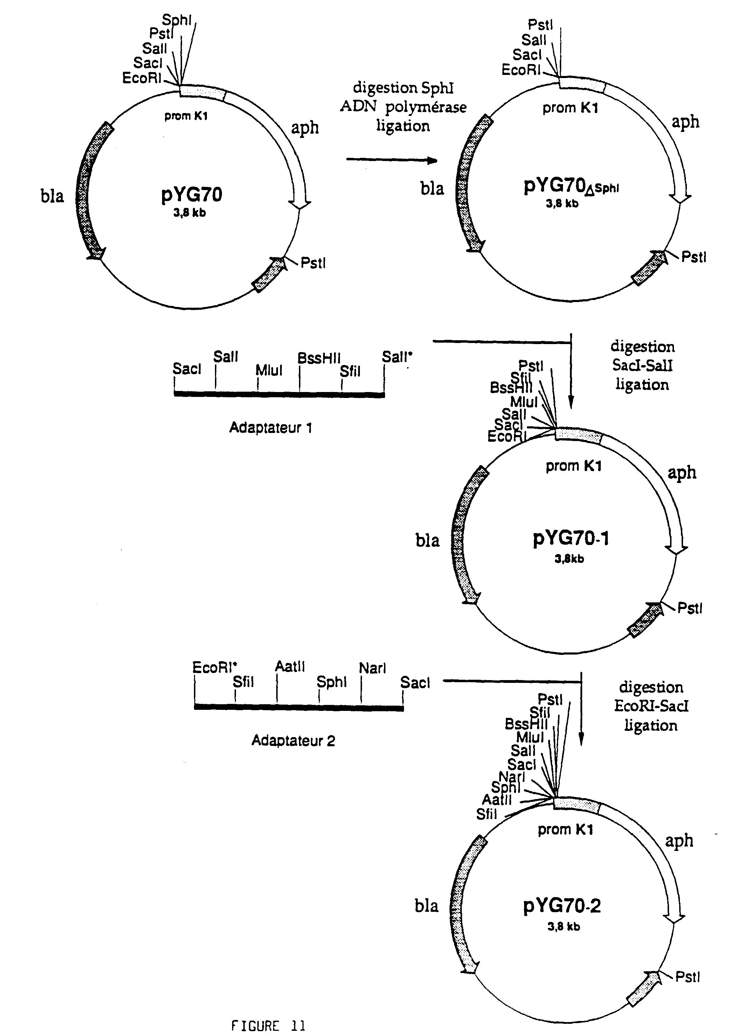

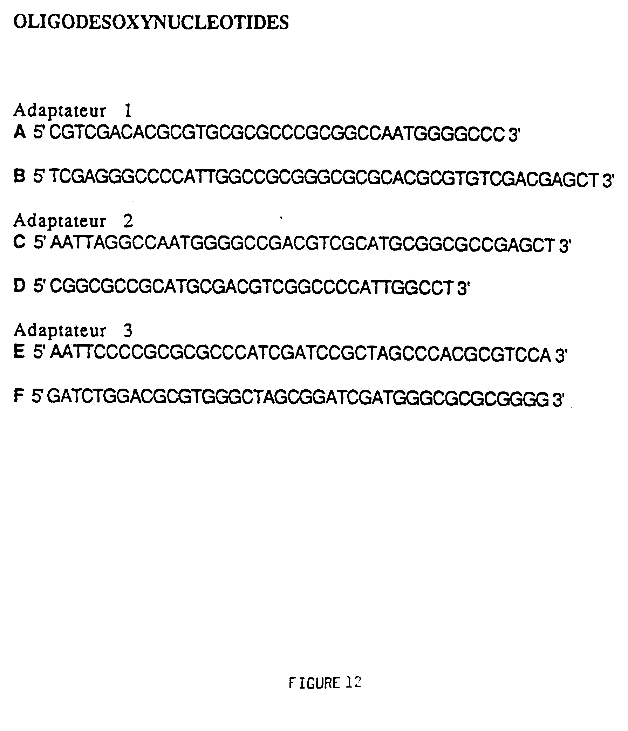

3.1. Isolement du gène PGK de K.lactis.
   Le gène PGK a été isolé chez K.lactis CBS2359 par criblage d'une banque génomique partielle avec une sonde hétérologue correspondant à la partie N-terminale du gène PGK de S.cerevisiae (Dobson et al., Nucl.Acid.Res. 10 (1982) 2625-2637). Plus précisément, la sonde utilisée correspond au fragment PvuI-EcoRI de 1,3 kb.
   En Southern Blot (Southern et al., J.Biol.Chem. 98 (1975) 503), la sonde utilisée s'hybride notamment à une séquence d'ADN contenue dans un fragment HindIII-HindIII de 4 kb. Cette séquence a été isolée par hybridation sur colonies au moyen de la sonde précédente. Pour cela, une banque restreinte d'ADN génomique de la souche CBS2359, constituée de fragments HindIII d'une taille comprise entre 3 et 5 kb introduits au site HindIII du plasmide pUC18, a été préparée et criblée.
   Un clone portant le plasmide pYG600 (figure 10) a ainsi été isolé. La séquence du gène PGK porté par ce plasmide a été décrite par Fournier et al. (Nucl.Acid.Res. 18 (1990) 369).
3.2. Construction d'un vecteur d'expression de la sérum-albumine humaine portant le gène PGK de K.lactis.
   Le plasmide pYG70 décrit sur la figure 5 a été modifié comme suit.
   (a) Modification des sites de restriction du plasmide pYG70 (figure 11).
      Pour faciliter les étapes ultérieures de clonage, certains sites de restriction ont été supprimés (i) et 2 adaptateurs ont été ajoutés (ii et iii) sur le plasmide pYG70.
      (i) Elimination du site SphI.
         Le plasmide pYG70 a été digéré par SphI, et les extrémités cohésives on ensuite été éliminées par digestion en présence d'ADN polymérase du phage T4. Après ligation en présence de ligase, le plasmide pYG70ΔSphI a été obtenu (voir figure 11).
      (ii) Insertion de l'adaptateur 1.
         L'adaptateur 1 a été obtenu par hybridation des oligodésoxynucléotides de synthèse A et B présentés sur la figure 12. Pour cela, 2 µg de chaque oligodésoxynucléotide ont été incubés dans un tampon d'hybridation qsp 20µl (tampon Tris-HCl 30 mM pH 7,5; NaCl 30 mmi,MgCl₂ 7,5 mM; ATP 0,25 mM; DTT 2 mM; EDTA 0,2 mM), puis la température a été élevée à 80°C pendant 10 minutes, et ramenée lentement à température ambiante.
         L'adaptateur ainsi obtenu contient des sites de coupure pour les enzymes suivantes : SacI; SalI; MluI; BssHII et SfiI, et permet d'éliminer, lors de son introduction, le site SalI présent sur le plasmide pYG70ΔSphI. Cet adaptateur a été introduit par ligation dans le plasmide pYG70ΔSphI préalablement digéré par les enzymes SalI et SacI.
         Le plasmide obtenu est appelé pYG70-1.
      (iii) Insertion de l'adaptateur 2.
         L'adaptateur 2 a été réalisé en suivant le protocole décrit pour l'adaptateur 1, en utilisant les oligodésoxynucléotides C et D décrits sur la figure 12. Cet adaptateur contient des sites de coupure pour les enzymes suivantes : SfiI; AatII; SphI; NarI et SacI et permet d'éliminer, lors de son introduction, le site EcoRI présent sur le plasmide pYG70-1. Il a été introduit par ligation dans le plasmide pYG70-1 préalablement digéré par les enzymes EcoRI et SacI, pour former le plasmide pYG70-2 (figure 11).
   (b) Introduction d'une cassette d'expression de la sérum-albumine humaine.
      La cassette d'expression de la sérum-albumine humaine utilisée comprend:
      - le promoteur inductible du gène LAC4 de K.lactis,
      - le gène de structure codant pour la sérum-albumine humaine (forme prépro), et
      - le terminateur du gène PGK de S.cerevisiae.

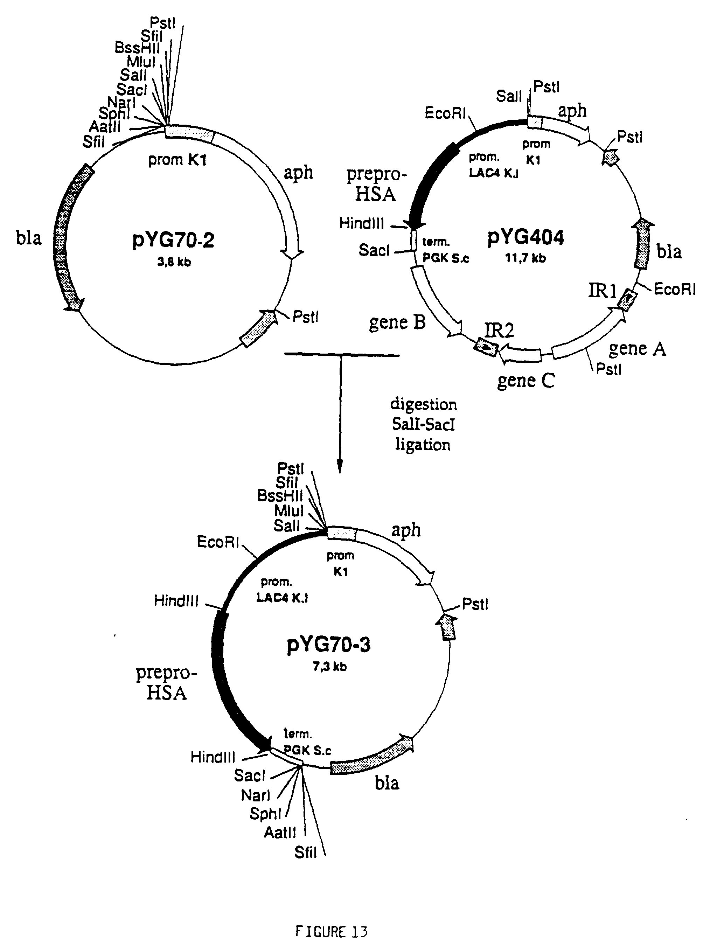

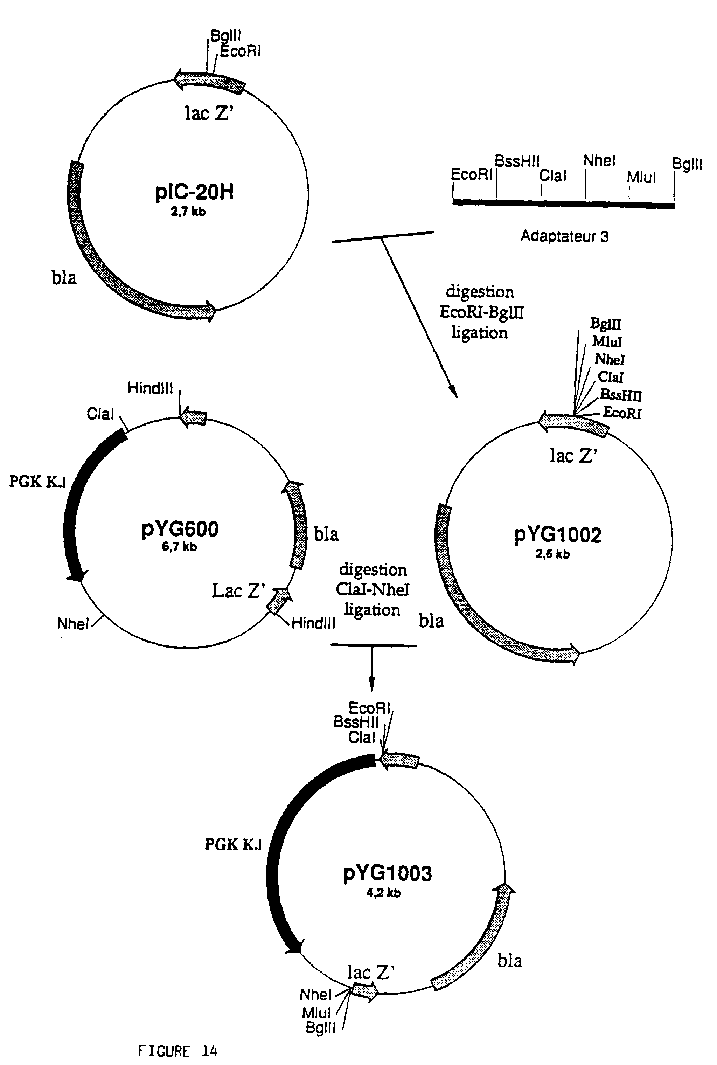

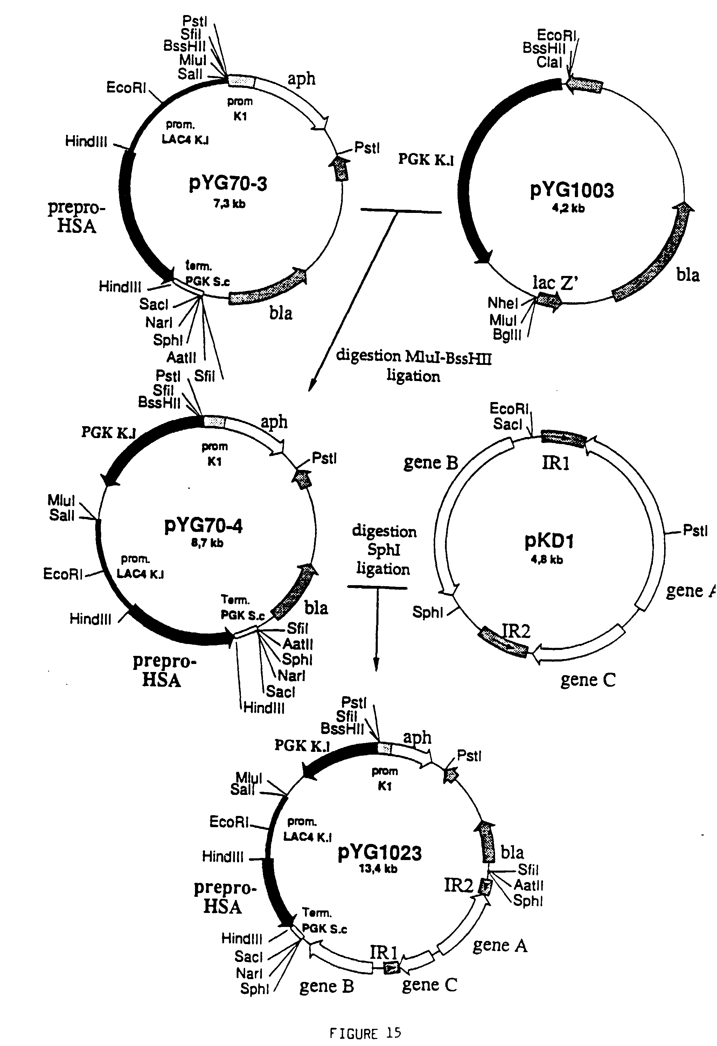

      Cette cassette a été isolée du plasmide pYG404 (EP361 991) sous forme d'un fragment SalI-SacI, puis introduite par ligation dans le plasmide pYG70-2 préalablement digéré par les enzymes correspondantes.
      Le plasmide obtenu est appelé pYG70-3 (figure 13).
   (c) Insertion du gène PGK de K.lactis.
      Le gène PGK K.lactis a été isolé du plasmide pYG600 (figure 10), sous-cloné dans le plasmide pYG1002 pour générer le plasmide pYG1003, puis isolé de ce dernier sous forme d'un fragment MluI-BssHII.
      Le sous-clonage dans pYG1002 a permis d'obtenir le gène PGK K.lactis dépourvu de son promoteur, et sous forme d'un fragment MluI-BssHII.
      Le plasmide pYG1003 a été obtenu de la manière suivante (figure 14):
      Le plasmide pIC20H (Marsh et al., Gene 32 (1984) 481) a été digéré par BglII et EcoRI de façon à introduire l'adaptateur 3. Cet adaptateur, qui apporte les sites EcoRI, BssHII, ClaI, NheI, MluI et BglII a été obtenu comme décrit précédemment (2.(a)(ii)), par hybridation des oligodésoxynucléotides E et F (figure 12). Le plasmide résultant est appelé pYG1002. Le gène PGK de K.lactis a été introduit dans ce nouveau plasmide sous forme d'un fragment ClaI-NheI, issu du plasmide pYG600. Le plasmide obtenu est appelé pYG1003 (figure 14).
      Le fragment MluI-BssHII issu du plasmide pYG1003 portant le gène PGK de K.lactis a ensuite été introduit dans les sites correspondants sur le plasmide pYG70-3, pour générer le plasmide pYG70-4 (figure 15).
      Sur ce plasmide, le gène PGK de K.lactis est désormais placé sous le contrôle du promoteur bidirectionnel k1 de la toxine Killer.
   (d) Insertion du réplicon levure.
      Les plasmides pYG70-4 (figure 15) et pKD1 (EP 361 991) ont été digérés par SphI et ligués ensemble en présence de ligase. A l'issue de cette ligation, 4 vecteurs peuvent être obtenus, selon la conformation du plasmide pKD1 (forme A ou forme B) et l'orientation de la partie correspondant au plasmide pYG70-4 par rapport à pKD1.
      L'une de ces constructions à été sélectionnée et appelé pYG1023 (figure 15). Ce vecteur comprend :
      - la séquence entière du plasmide pKD1, qui fait de pYG1023 un plasmide à copies multiples, stable, et capable de se répliquer chez les levures et préférentiellement les levures du genre Kluyveromyces,
      - une cassette d'expression de la sérum-albumine humaine comportant le gène de structure codant pour la forme prépro sous contrôle du promoteur inductible du gène LAC4 de K.lactis, et du terminateur du gène PGK de S.cerevisiae,
      - un réplicon et un marqueur de sélection (gène bla conférant la résistance à l'ampicilline) pour E.coli,
      - deux marqueurs de sélection pour une souche K.lactis pgk : le gène aph muté sous contrôle du promoteur bidirectionnel kl de la toxine Killer et le gène PGK de K.lactis sous contrôle du même promoteur mais transcrit de façon divergente par rapport au gène aph·

### B - Production par voie recombinante de sérum-albumine humaine

**B1.** Cet exemple décrit un procédé classique de production de SAH recombinante utilisant la levure K.lactis CBS683 transformée par le vecteur pYG401 (exemple A1). Cet exemple illustre l'art antérieur.

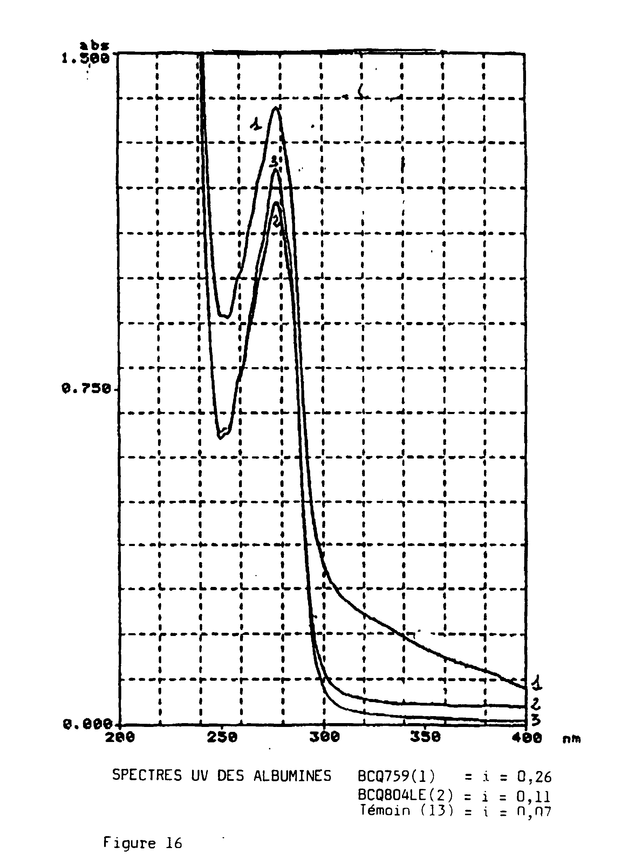

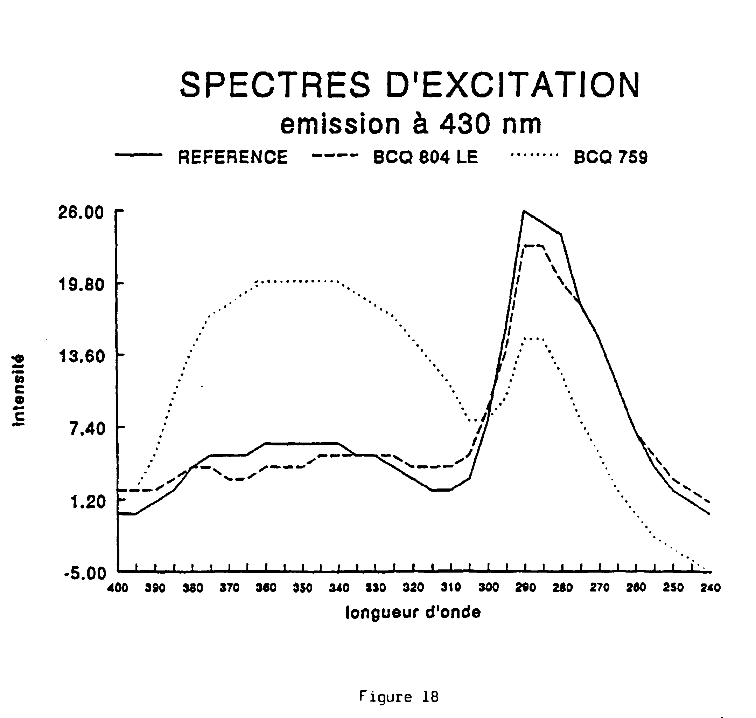

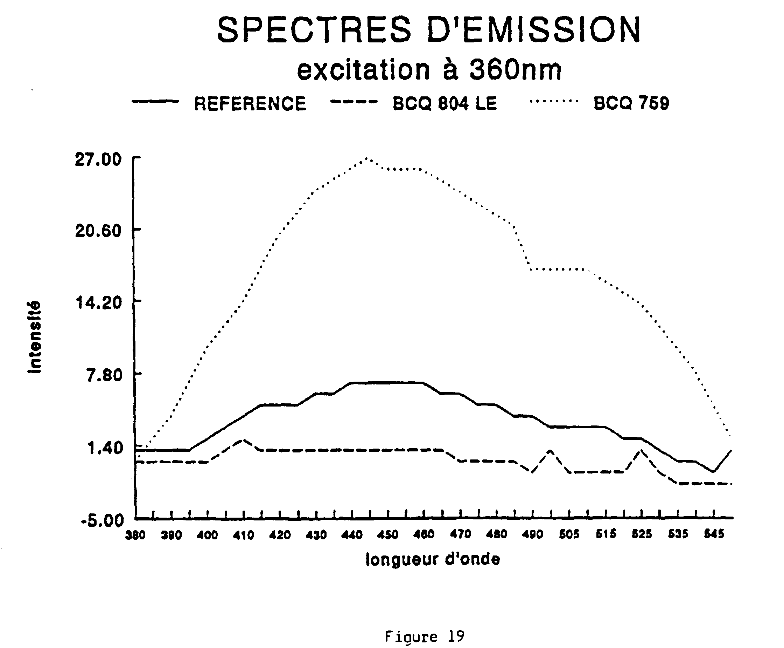

La culture a été réalisée en fermenteur de 2 litres à 28°C en mode fed-batch. Initialement, le fermenteur contient 0,7 litre de milieu de base (glucose 2 g/l, extrait de levure 3 g/l, sels). Une culture de 50 ml en phase exponentielle de croissance (inoculée à partir d'une suspension congelée) est utilisée pour inoculer le fermenteur. Après une phase préliminaire de croissance de type batch, la charge complémentaire (glucose, corn steep, sels d'ammonium : 40%/13%/7% [w/v]) est ajoutée exponentiellement. Après 64 heures de culture, le moût est centrifugé et le surnageant microfiltré sur membrane 2µ. Le protocole de purification comprend une étape de chromatographie d'affinité sur Bleu Trisacryl (IBF, France) d'où l'albumine est éluée par une forte concentration saline (NaCl 3,5 M), puis, après concentration et diafiltration, un passage sur échangeur d'ion de type Q-sépharose "fast flow". La SAH ainsi purifiée présente une bande homogène en électrophorèse SDS-PAGE et ne se distingue pas d'albumines naturelles prises comme référence dans de nombreux tests de caractérisation biochimique. Pourtant, une solution de la SAH obtenue présente une coloration jaune avec un indice de colorimétrie i = 0,26 qu'il n'est pas possible d'éliminer par une purification plus poussée et/ou des traitements tels que l'utilisation de charbons activés, ainsi qu'une fluorescence anormale après excitation à 360 nm (voir figures 16, 18 et 19).

**B2.** Un lot d'albumine recombinante BCQ 759 obtenu dans les conditions décrites dans l'exemple **B1** présente un indice de coloration i = 0,26 après purification (figure 16). De nombreux essais de chromatographie supplémentaires et de traitements physicochimiques ne permettent pas de diminuer cette coloration.

Afin d'analyser la nature de la liaison de cette coloration à l'albumine, un cycle de dénaturation-renaturation a été réalisé comme suit :

50 mg de BCQ 759 sont dénaturés par incubation dans 2 ml de guanidine-HCl 7M et 0,3M de β-mercaptoéthanol. Cette solution est chauffée 1 heure à 100°C. Après refroidissement, 500 µl de rSAH dénaturée sont injectés sur une colonne TSK 3000SW équilibrée avec de l'urée 8M et 0,3M de β-mercaptoéthanol pour éliminer les petites molécules qui pourraient être fixées fortement (mais non covalemment) à la SAH. Les fractions absorbant à 280 nm sont dialysées contre une solution Tris HCl 50 mM, urée 8M, pH 8,0 pendant une nuit sous azote puis contre 1 l de tampon de renaturation Tris HCl 50 mM, pH 9,5 contenant 50 mM NaCl, 1 mM de glutathion réduit et 0,1 mM de glutathion oxydé sous azote pendant 48 heures. Après dialyse finale contre de l'eau, un UV est réalisé et l'on calcule un indice de coloration i₂ = 0,22. Dans les mêmes conditions une albumine placentaire témoin (Institut Mérieux, i = 0,07) donne après renaturation i₂ = 0,14. Cet exemple met clairement en évidence la nature essentiellement covalente de la liaison du pigment à l'albumine. Il est donc vain d'essayer de décolorer significativement une albumine recombinante par le procédé de purification.

**B3.** Dans cet exemple est décrite une expérience simple qui permet de mettre en évidence que la source de carbone est une cause essentielle du développement de la coloration.

L'albumine témoin (i = 0,07) est incubée dans différents milieux de culture en erlen sans inoculation par la levure. Les milieux testés sont constitués d'un milieu de base Bo défini comme suit :

| | | |
|---|---|---|
| Sels | Acétate d'ammonium | 7 g/l |
| | Na₂HPO₄ | 4,4 g/l |
| | KH₂PO₄ | 4 g/l |
| | MgSO₄, 7H₂O | 0,5 g/l |
| | CaCl₂, 2H₂O | 0,1 g/l |
| | MnSO_{4,} H₂O | 10 mg/l |
| | ZnSO_{4,} 7H₂O | 100 mg/l |
| | FeSO₄,7H₂O | 15 mg/l |
| | AlCl_{3,} 6H₂O | 1 mg/l |
| | CoCl_{2,} 6H₂O | 2 mg/l |
| | CuSO_{4,} 5H₂O | 0,13 mg/l |
| | KI | 0,33 mg/l |
| | H₃BO₃ | 1,47 mg/l |
| | Na₂MoO₄ | 0,67 mg/l |

| | | |
|---|---|---|
| Vitamines | Mésoinositol | 2 mg/l |
| | Acide nicotinique | 2 mg/l |
| | Biotine | 2 mg/l |
| | Pantothénate de calcium | 2 mg/l |

| | | |
|---|---|---|
| Acides aminés | L-Glu | 0,6 g/l |
| | L-Phe | 0,15 g/l |
| | DL-Ala | 0,7 g/l |
| | L-Leu | 0,35 g/l |
| | L-Lys, HCI | 0,30 g/l |
| | DL-His, HCI | 0,30 g/l |
| | L-Ile | 0,10 g/l |
| | DL-Met | 0,15 g/l |
| | L-Pro | 0.3 g/l |

et d'une source de carbone :

| | | | |
|---|---|---|---|
| erlen | E1 | Glucose (microfiltré) | 20 g/l |
| | E2 | Lactose | 20 g/l |
| | E3 | contrôle (sans source de carbone) | |

Les trois erlens sont incubés avec HSA à 50 mg/l et agités à 28°C pendant 3 jours.

Après 3 jours, le milieu est centrifugé et filtré sur une membrane 0,2µ. On ajoute 5 ml de support d'affinité Bleu Trisacryl M (IBF France) et on agite doucement. Après 1 heure, les eaux-mères sont séparées sur verre fritté et l'albumine éluée par une solution NaCl 3,5M. L'éluat est ensuite concentré et diafiltré par de l'eau sur membrane d'ultrafiltration 10 kD.

Une analyse spectrophotométrique donne les résultats suivants pour l'indice de coloration de l'albumine :

| | | |
|---|---|---|
| erlen | E1 | i = 0,16 |
| | E2 | i = 0,12 |
| | E3 | i = 0,09 |
| | SAH témoin | i = 0,07 |

Cet exemple montre donc clairement que la source de carbone induit une augmentation de la coloration de l'albumine dans les conditions de culture sans levure.

**B4**. Pour compléter les résultats obtenus dans l'exemple précédent, l'albumine de référence a été incubée dans les mêmes conditions en présence de :

| | | | |
|---|---|---|---|
| erlen | E4 | milieu Bo + glucose autoclavé | 20 g/l |
| | E5 | milieu Bo + glucose microfiltré | 20 g/l |
| | E6 | milieu Bo + saccharose microfiltré | 20 g/l |

Après concentration et diafiltration du milieu pour éliminer les petites molécules, l'indice de coloration a été déterminé en UV :

| | |
|---|---|
| E4 | i = 0,22 |
| E5 | i = 0,15 |
| E6 | i=0,14 |

**B5.** Afin de vérifier les conclusions obtenues dans les exemples précédents lors d'une culture réelle, la coloration de l'albumine recombinante produite en erlen par la levure K.lactis a été analysée.
a) Souche K.lactis pgk⁻ CBS 295.91 transformée par le vecteur pYG1023 décrit dans l'exemple A3 :

| | | |
|---|---|---|
| Erlens | E7 = | Bo + lactose 10 g/l + éthanol 10 g/l |
| | E8 = | Bo + lactose 20 g/l |
| | E9 = | Bo + glucose microfiltré 20 g/l |
| | E10 = | Bo + lactose 10 g/l + glucose 10 g/l |

L'albumine recombinante produite après 72 heures de culture en mode batch en erlenmeyers de 250 ml à 28°C est purifiée sur colonne d'échange d'anions Mono Q HR5/5 (Pharmacia) et traitée au charbon activé pour éliminer la coloration qui n'est pas fixée de manière covalente à la protéine. Les résultats obtenus après analyse UV sont les suivants :

| | | |
|---|---|---|
| E7 | i = 0,11 | BCQ 804 LE (voir figures 16, 18 et 19) |
| E8 | i = 0,14 | BCQ 804 L |
| E9 | i = 0,17 | BCQ 804 G |
| E10 | i = 0,15 | BCQ 804 LG |

b) Souche K.lactis CBS 293.91 transformée par le vecteur pYG132 décrit dans l'exemple A2:

| | | |
|---|---|---|
| Erlen: | E11 = | Bo + glycérol 10 g/l + éthanol 10 g/l |

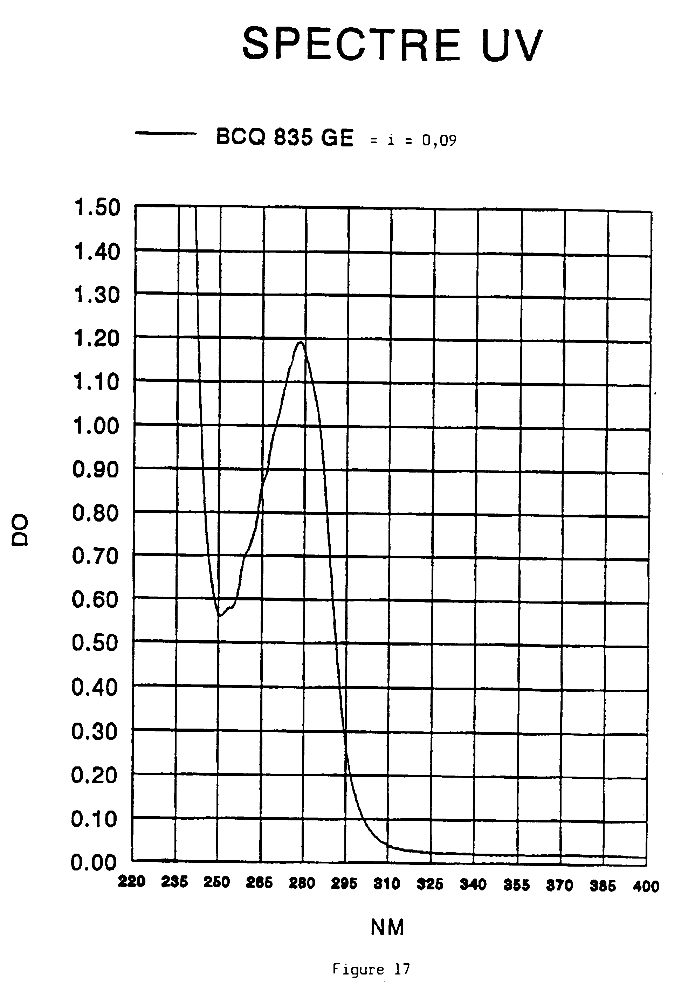

Dans les mêmes conditions que précédemment, l'albumine recombinante produite donne après simple purification sur Mono Q un indice de coloration i = 0,09 (BCQ 835 GE) : voir figure 17.

## Revendications

1. Sérum-albumine humaine résultant de l'expression, dans une levure du genre Kluyveromyces, d'une séquence d'ADN exogène et possédant un indice de colorimétrie inférieur à 0,2, susceptible d'être obtenue par la mise en oeuvre du procédé de préparation de ladite sérum-albumine humaine comprenant les étapes suivantes :
- dans une première étape, on introduit dans une cellule hôte un ADN exogène codant pour la sérum-albumine humaine sous contrôle de signaux de transcription et de traduction appropriés à l'hôte utilisé,
- dans une deuxième étape, on cultive la cellule ainsi obtenue dans un milieu de composition définie contenant au moins une source carbonée choisie parmi les alcools, les sucres non-réducteurs, les acides organiques et les dérivés du glucose substitués sur l'oxygène du carbone C4 ; ou dans un milieu préparé de façon à éliminer ou à limiter la formation d'impuretés de type aldéhydique, et,
- dans une troisième étape, on récupère la SAH produite.

2. Sérum-albumine humaine selon la revendication 1 caractérisée en ce qu'elle possède un indice de colorimétrie inférieur à 0,15.

3. Sérum-albumine humaine selon la revendication 2 caractérisée en ce qu'elle possède un indice de colorimétrie inférieur à 0,1.

4. Sérum-albumine humaine selon l'une quelconque des revendications 1 à 3 caractérisée en ce que l'ADN exogène est choisi parmi les séquences d'ADNc, les séquences d'ADN génomique et les séquences hybrides codant pour la SAH.

5. Sérum-albumine humaine selon la revendication 4 caractérisée en ce que l'ADN exogène comprend une région de démarrage de la transcription et de la traduction jointe à l'extrémité 5' de la séquence codant pour la SAH.

6. Sérum-albumine humaine selon l'une quelconque des revendications 1 à 5 caractérisée en ce que l'ADN exogène fait partie d'un vecteur, qui peut être à réplication autonome ou intégratif.

7. Sérum-albumine humaine selon l'une quelconque des revendications 1 à 6 caractérisée en ce qu'elle résulte de l'expression dans une levure du genre Kluyveromyces, d'une séquence d'ADN exogène et de la sécrétion du produit d'expression de ladite séquence dans le milieu de culture.

8. Sérum-albumine humaine selon la revendication 7 caractérisée en ce que la séquence d'ADN exogène comprend, en amont de la séquence codant pour la SAH mature, une séquence "leader" dirigeant la protéine naissante dans les voies de sécrétion de l'hôte utilisé.

9. Sérum-albumine humaine selon la revendication 8, caractérisée en ce que la séquence d'ADN exogène comprend, dans la direction 5' - 3', une région de démarrage de la transcription et de la traduction, une séquence "leader" dirigeant la protéine naissante dans les voies de sécrétion de la cellule hôte utilisée et un ADNc codant pour la sérum-albumine humaine.

10. Sérum-albumine humaine selon l'une des revendications 8 ou 9, caractérisée en ce que la séquence "leader" peut être la séquence "leader" naturelle de la SAH, une séquence "leader" hétérologue, ou une séquence "leader" artificielle.

11. Procédé de préparation de Sérum-albumine humaine ayant un indice de colorimétrie inférieur à 0,2 caractérisé en ce que l'on effectue les étapes suivantes :
- dans une première étape, on introduit dans une cellule hôte eucaryote ou procaryote un ADN exogène codant pour la sérum-albumine humaine sous contrôle de signaux de transcription et de traduction appropriés à l'hôte utilisé,
- dans une deuxième étape, on cultive la cellule ainsi obtenue dans un milieu de composition définie contenant au moins une source carbonée choisie parmi les alcools, les sucres non-réducteurs, les acides organiques et les dérivés du glucose substitués sur l'oxygène du carbone C4; ou dans un milieu préparé de façon à éliminer ou à limiter la formation d'impuretés de type aldéhydique,et,
- dans une troisième étape, on récupère la SAH produite.

12. Procédé selon la revendication 11 caractérisé en ce que l'ADN exogène est défini comme dans l'une des revendications 4, 5, 6, 8, 9 ou 10.

13. Procédé selon la revendication 12 caractérisé en ce que la SAH est sécrétée dans le milieu de culture.

14. Procédé selon la revendication 11 caractérisé en ce que l'hôte eucaryote est choisi parmi les cellules animales, les levures et les champignons.

15. Procédé selon la revendication 11 caractérisé en ce que l'hôte procaryote est choisi parmi les bactéries E.coli, Bacillus et Streptomyces.

16. Procédé selon la revendication 14 caractérisé en ce que l'hôte est choisi parmi les levures du genre Saccharomyces, Kluyveromyces, Pichia, Schwanniomyces et Hansenula.

17. Procédé selon la revendication 16 caractérisé en ce que l'hôte est choisi parmi les levures du genre Kluyveromyces.

18. Procédé selon la revendication 11 caractérisé en ce que l'alcool peut être choisi parmi les alcools simples comportant au moins 2 atomes de carbone et les polyalcools.

19. Procédé selon la revendication 11 caractérisé en ce que les dérivés du glucose sont choisis parmi les disaccharides, et de préférence parmi les disaccharides présentant une liaison osidique de type 1-4.

20. Procédé selon la revendication 11 caractérisé en ce que le milieu comprend une combinaison de plusieurs sources carbonées.

21. Procédé selon la revendication 20 caractérisé en ce que la source carbonée est composée de l'association d'un dérivé du glucose et d'un alcool, ou de l'association d'un alcool simple et d'un polyalcool.

22. _ Procédé selon la revendication 11 caractérisé en ce que le milieu de culture est préalablement stérilisé à froid.

23. Composition pharmaceutique comprenant de la SAH selon l'une quelconque des revendications 1 à 10.

## Claims

1. Human serum albumin resulting from the expression, in a yeast of the genus Kluyveromyces, of an exogenous DNA sequence and possessing a colorimetric index of less than 0.2, capable of being obtained using the process for preparing the said human serum albumin comprising the following steps:
- in a first step, an exogenous DNA encoding human serum albumin under the control of transcriptional and translational signals appropriate to the host used is introduced into a host cell,
- in a second step, the cell thus obtained is cultured in a medium of defined composition containing at least one carbon source chosen from alcohols, nonreducing sugars, organic acids and glucose derivatives substituted on the oxygen of the carbon C4; or in a medium prepared so as to remove or limit the formation of aldehyde type impurities, and,
- in a third step, the HSA produced is recovered.

2. Human serum albumin according to Claim 1, characterized in that it has a colorimetry index of less than 0.15.

3. Human serum albumin according to Claim 2, characterized in that it has a colorimetry index of less than 0.1.

4. Human serum albumin according to any one of Claims 1 to 3, characterized in that the exogenous DNA is chosen from the cDNA sequences, the genomic DNA sequences and the hybrid sequences encoding HSA.

5. Human serum albumin according to Claim 4, characterized in that the exogenous DNA comprises a region for initiation of transcription and translation joined to the 5' end of the sequence encoding HSA.

6. Human serum albumin according to any one of Claims 1 to 5, characterized in that the exogenous DNA is part of a vector, which may be one affording autonomous or integrative replication.

7. Human serum albumin according to any one of Claims 1 to 6, characterized in that it results from the expression, in a yeast of the genus Kluyveromyces, of an exogenous DNA sequence and from the secretion of the product of expression of the said sequence into the culture medium.

8. Human serum albumin according to Claim 7, characterized in that the exogenous DNA sequence comprises, upstream of the sequence encoding the mature HSA, a "leader" sequence directing the nascent protein in the secretory pathways of the host used.

9. Human serum albumin according to Claim 8, characterized in that the exogenous DNA sequence comprises, in the 5'-3' direction, a region for initiation of transcription and translation, a "leader" sequence directing the nascent protein in the secretory pathways of the host cell used, and a cDNA encoding human serum albumin.

10. Human serum albumin according in either of Claims 8 and 9, characterized in that the "leader" sequence may be the natural "leader" sequence of HSA, a heterologous "leader" sequence, or an artificial "leader" sequence.

11. Process for preparing human serum albumin having a colorimetry index of less than 0.2, characterized in that the following steps are performed:
- in a first step, an exogenous DNA encoding human serum albumin under the control of transcriptional and translational signals appropriate to the host used is introduced into a eucaryotic or procaryotic host cell,
- in a second step, the cell thus obtained is cultured in a medium of defined composition containing at least one carbon source chosen from alcohols, non-reducing sugars, organic acids and glucose derivatives substituted on the oxygen the carbon C4; or in a medium prepared so as to remove or limit the formation of aldehyde type impurities, and,
- in a third step, the HSA produced is recovered.

12. Process according to Claim 11, characterized in that the exogenous DNA is defined as in one of Claims 4, 5, 6, 8, 9 or 10.

13. Process according to Claim 12, characterized in that the HSA is secreted into the culture medium.

14. Process according to Claim 11, characterized in that the eucaryotic host is chosen from animal cells, yeasts and fungi.

15. Process according to Claim 11, characterized in that the procaryotic host is chosen from the bacteria E. coli, Bacillus and Streptomyces.

16. Process according to Claim 14, characterized in that the host is chosen from yeasts of the genus Saccharomyces, Kluyveromyces, Pichia, Schwanniomyces and Hansenula.

17. Process according to Claim 16, characterized in that the host is chosen from the yeasts of the genus Kluyveromyces.

18. Process according to Claim 11, characterized in that the alcohol may, be chosen from simple alcohols containing at least two carbon atoms and polyalcohols.

19. Process according to Claim 11, characterized in that the glucose derivatives are chosen from disaccharides, and preferably from the disaccharides having a 1-4 type glycoside bond.

20. Process according to Claim 11, characterized in that the medium comprises a combination of several carbon sources.

21. Process according to Claim 20, characterized in that the carbon source is composed of the combination of a glucose derivative and an alcohol, or the combination of a simple alcohol and a polyalcohol.

22. Process according to Claim 11, characterized in that the culture medium is previousy sterilized at cold temperature.

23. Pharmaceutical composition comprising HSA according to any one of Claims 1 to 10.

## Patentansprüche

1. Humanserumalbumin als Ergebnis der Expression einer exogenen DNA-Sequenz in einer Hefe der Gattung Kluyveromyces, das einen Kolorimetrieindex von kleiner 0,2 besitzt, und durch Durchführen des Verfahrens zur Herstellung des Humanserumalbumins erhalten werden kann, das die folgenden Stufen umfaßt:
- in einer ersten Stufe wird in eine Wirtszelle eine exogene DNA, die Humanserumalbumin unter der Kontrolle von für den verwendeten Wirt geeigneten Transkriptionsund Translationssignalen codiert, eingebracht,
- in einer zweiten Stufe wird die so erhaltene Zelle in einem Medium mit definierter Zusammensetzung, das mindestens eine Kohlenstoffquelle, ausgewählt aus Alkoholen, nichtreduzierenden Zuckern, organischen Säuren und Glucosederivaten, die am Sauerstoff des Kohlenstoffatoms C4 substituiert sind, enthält, oder in einem Medium, das so hergestellt wurde, daß die Bildung von Verunreinigungen vom Aldehydtyp beseitigt oder beschränkt ist, gezüchtet, und
- in einer dritten Stufe wird das gebildete HSA isoliert.

2. Humanserumalbumin nach Anspruch 1, dadurch **gekennzeichnet,** daß es einen Kolorimetrieindex von kleiner 0,15 besitzt.

3. Humanserumalbumin nach Anspruch 2, dadurch **gekennzeichnet,** daß es einen Kolorimetrieindex von kleiner 0,1 besitzt.

4. Humanserumalbumin nach einem der Ansprüche 1 bis 3, dadurch **gekennzeichnet,** daß die exogene DNA aus cDNA-Sequenzen, genomischen DNA-Sequenzen und Hybridsequenzen, die HSA codieren, ausgewählt ist.

5. Humanserumalbumin nach Anspruch 4, dadurch **gekennzeichnet,** daß die exogene DNA eine Transkriptionsstartregion in der Nähe des 5'-Endes der Sequenz, die HSA codiert, besitzt.

6. Humanserumalbumin nach einem der Ansprüche 1 bis 5, dadurch **gekennzeichnet,** daß die exogene DNA Teil eines Vektors ist, der sich autonom oder integrativ replizieren kann.

7. Humanserumalbumin nach einem der Ansprüche 1 bis 6, dadurch **gekennzeichnet,** daß er das Ergebnis der Expression einer exogenen DNA-Sequenz in einer Hefe der Gattung Kluyveromyces und der Sekretion des Expressionsprodukts der Sequenz in das Kulturmedium ist.

8. Humanserumalbumin nach Anspruch 7, dadurch**gekennzeichnet,** daß die exogene DNA stromaufwärts der Sequenz, die für reifes HSA codiert, eine Leader -Sequenz umfaßt, die das entstehende Protein in die Sekretionswege des verwendeten Wirts steuert.

9. Humanserumalbumin nach Anspruch 8, dadurch **gekennzeichnet,** daß die exogene DNA-Sequenz in der 5'-3'-Richtung eine Startregion der Transkription und der Translation, eine Leader-Sequenz, die das entstehende Protein in die Sekretionswege der verwendeten Wirtszelle steuert, und eine cDNA, die Humanserumalbumin codiert, umfaßt.

10. Humanserumalbumin nach einem der Ansprüche 8 oder 9, dadurch **gekennzeichnet,** daß die Leader-Sequenz die natürliche Leader-Sequenz von HSA, eine heterologe Leader-Sequenz oder eine künstliche Leader-Sequenz sein kann.

11. Verfahren zur Herstellung von Humanserumalbumin mit einem Kolorimetrieindex von kleiner 0,2, dadurch **gekennzeichnet,** daß man die folgenden Stufen durchführt:
- in einer ersten Stufe wird in eine eukaryontische oder prokaryontische Wirtszelle eine exogene DNA, die Humanserumalbumin unter der Kontrolle von für den verwendeten Wirt geeigneten Transkriptions- und Translationssignalen codiert, eingebracht,
- in einer zweiten Stufe wird die so erhaltene Zelle in einem Medium mit definierter Zusammensetzung, das mindestens eine Kohlenstoffquelle, ausgewählt aus Alkoholen, nichtreduzierenden Zuckern, organischen Säuren und Glucosederivaten, die am Sauerstoff des Kohlenstoffs C4 substituiert sind, enthält, oder in einem Medium, das so hergestellt wurde, daß die Bildung von Verunreinigungen vom Aldehydtyp beseitigt oder begrenzt ist, gezüchtet und
- in einer dritten Stufe wird das gebildete HSA isoliert.

12. Verfahren nach Anspruch 11, dadurch **gekennzeichnet,** daß die exogene DNA wie in einem der Ansprüche 4, 5, 6, 8, 9 oder 10 definiert ist.

13. Verfahren nach Anspruch 12, dadurch **gekennzeichnet,** daß das HSA in das Kulturmedium sezerniert wird.

14. Verfahren nach Anspruch 11, dadurch **gekennzeichnet,** daß der eukaryontische Wirt aus tierischen Zellen, Hefen und Pilzen ausgewählt wird.

15. Verfahren nach Anspruch 11, dadurch **gekennzeichnet,** daß der prokaryontische Wirt aus den Bakterien E. coli, Bacillus und Streptomyces ausgewählt wird.

16. Verfahren nach Anspruch 14, dadurch **gekennzeichnet,** daß der Wirt aus den Hefen der Gattung Saccharomyces, Kluyveromyces, Pichia, Schwanniomyces und Hansenula ausgewählt wird.

17. Verfahren nach Anspruch 16, dadurch **gekennzeichnet,** daß der Wirt aus den Hefen der Gattung Kluyveromyces ausgewählt wird.

18. Verfahren nach Anspruch 11, dadurch **gekennzeichnet,** daß der Alkohol aus einfachen Alkoholen, die mindestens zwei Kohlenstoffatome enthalten, und Polyalkoholen ausgewählt werden kann.

19. Verfahren nach Anspruch 11, dadurch **gekennzeichnet,** daß die Glucosederivate aus Disacchariden und bevorzugt aus Disacchariden, die eine Osidbindung vom 1-4-Typ besitzen, ausgewählt werden.

20. Verfahren nach Anspruch 11, dadurch **gekennzeichnet,** daß das Medium eine Kombination aus mehreren Kohlenstoffquellen umfaßt.

21. Verfahren nach Anspruch 20, dadurch **gekennzeichnet,** daß die Kohlenstoffquelle aus der Assoziation eines Glucosederivats und eines Alkohols oder der Assoziation eines einfachen Alkohols und eines Polyalkohols zusammengesetzt ist.

22. Verfahren nach Anspruch 11, dadurch **gekennzeichnet,** daß das Kulturmedium zuvor in der Kälte sterilisiert wurde.

23. Pharmazeutische Zusammensetzung, umfassend das HSA nach einem der Ansprüche 1 bis 10.
